(19) Europäisches Patentamt
European Patent Office
Office européen des brevets

(11) Veröffentlichungsnummer: **0 243 868**
**A2**

(12)
# EUROPÄISCHE PATENTANMELDUNG

(21) Anmeldenummer: **87105908.5**

(22) Anmeldetag: **22.04.87**

(51) Int. Cl.³: **C 07 D 213/70**
C 07 D 213/84, C 07 D 405/1-2
C 07 D 401/12, A 01 N 43/10

(30) Priorität: **02.05.86 DE 3614846**

(43) Veröffentlichungstag der Anmeldung:
**04.11.87 Patentblatt 87/45**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Sasse, Klaus, Dr.**
**Pützweg 13**
**D-5060 Bergisch-Gladbach 2(DE)**

(72) Erfinder: **Fischer, Reiner, Dr.**
**Rembrandtstrasse 15**
**D-4019 Monheim(DE)**

(72) Erfinder: **Santel, Hans-Joachim, Dr.**
**Grünstrasse 9a**
**D-5090 Leverkusen 1(DE)**

(72) Erfinder: **Schmidt, Robert R., Dr.**
**Im Waldwinkel 110**
**D-5060 Bergisch-Gladbach 2(DE)**

(54) **Pyridylthio-acylanilide.**

(57) Die Erfindung betrifft Pyridylthio-acylanilide der Formel (I),

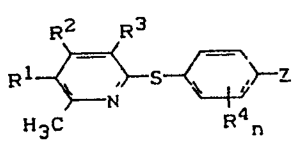

(I)

in welcher
Z für die Gruppe (Ia)

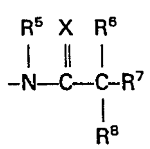

(Ia)

oder die Gruppe (Ib)

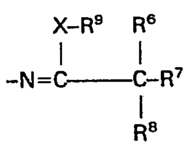

(Ib) steht,

und die restlichen Substituenten in der Beschreibung näher definiert sind,
mehrere Verfahren zu ihrer Herstellung und ihre Verwendung als Herbizide.

EP 0 243 868 A2

BAYER AKTIENGESELLSCHAFT
Konzernverwaltung RP
Patentabteilung

5090 Leverkusen, Bayerwerk

KM/Ke-c
Ib

# Pyridylthio-acylanilide

Die vorliegende Erfindung betrifft neue Pyridylthio-acylanilide, mehrere Verfahren zu ihrer Herstellung und ihre Verwendung als Herbizide.

Es ist bereits bekannt, daß bestimmte Carbonsäureanilide herbizide Eigenschaften besitzen (vgl. R. Wegler "Chemie der Pflanzenschutz- und Schädlingsbekämpfungsmittel" Bd. 2, Seiten 311-314, Springer-Verlag, Berlin 1970). So kann z.B. das Propionsäure-3,4-dichloranilid zur Unkrautbekämpfung eingesetzt werden. Die herbizide Wirkung dieser Verbindung gegenüber Unkräuter ist jedoch ebenso wie ihre Verträglichkeit gegenüber wichtigen Nutzpflanzen nicht immer in allen Anwendungsgebieten völlig zufriedenstellend.

Ferner ist bekannt, daß zahlreiche Pyrimidinyl-2-ether und -thioether als Herbizide geeignet sind (vgl. JP-OS 9 474/1967, US-PS 3 126 271 und US-PS 3 250 775). Z.B. können das 2-Phenoxy-4,6-dimethyl-pyrimidin und das 2-(4-Chlor-benzylthio)-4,6-dimethyl-pyrimidin zur Bekämpfung von Unkräutern verwendet werden. Die herbizide Potenz dieser Stoffe ist aber nicht immer ausreichend.

Weiterhin ist bekannt, daß niedere Acylderivate von 4-Pyridyloxy- (bzw. thio)-anilinen herbizide Eigenschaften aufweisen (vgl. DE-OS 2 501 648, JP-OS 55-122 763 und JP-OS 56 123 970). Darüberhinaus sind auch herbizid wirksame Acylderivate von 4-Pyrimidyloxy-anilinen bekannt, die in der 5-Stellung des Pyrimidylrestes durch Halogen oder Trifluormethyl substituiert sind, dagegen in den Positionen 4 und 6 keinen Substituenetn enthalten (vgl. JP-OS 56-029 576). Auch die Wirksamkeit dieser Stoffe ist jedoch nicht immer befriedigend.

Es wurden nun neue substituierte Carbonsäureanilide der Formel (I),

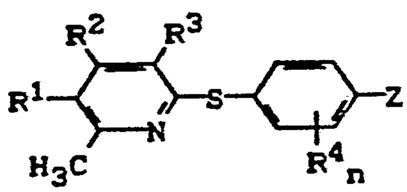

(I)

in welcher

$R^1$, $R^2$ und $R^3$ unabhängig voneinander für Wasserstoff, Halogen, Cyano, Trifluormethyl, für Alkyl, Alkoxy und Alkylthio mit jeweils 1 bis 4 Kohlenstoffatomen stehen,

$R^4$ für Halogen, Methyl oder Methoxy steht,

n für eine Zahl 0, 1 oder 2 steht,

Z für die Gruppe (Ia)

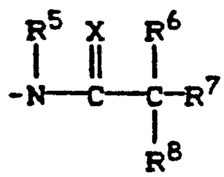

(Ia)

oder die Gruppe (Ib)

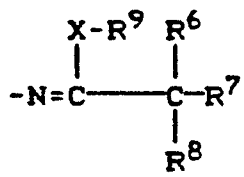

(Ib)

steht,

wobei

X für Sauerstoff, Schwefel, eine Gruppe $N-R^{10}$ oder $N-O-R^{11}$ steht, wobei

$R^{10}$ und $R^{11}$ unabhängig voneinander für Wasserstoff oder gegebenenfalls substituiertes Alkyl mit 1 bis 6 Kohlenstoffatomen, für Alkenyl oder Alkinyl mit jeweils 2 bis 6 Kohlenstoffatomen stehen,

$R^5$ für Wasserstoff oder gegebenenfalls substituiertes Alkyl mit 1 bis 6 Kohlenstoffatomen oder jeweils gegebenenfalls substituiertes Alkenyl oder Alkinyl mit jeweils 2 bis 6 Kohlenstoffatomen steht,

$R^6$ für Wasserstoff, Halogen, Cyano, für gegebenenfalls substituiertes Alkyl mit 1 bis 6 Kohlenstoffatomen, Alkenyl mit 2 bis 6 Kohlenstoffatomen, jeweils gegebenenfalls substituiertes Aryl oder Aralkyl oder für die Reste $-OR^{12}$ oder $-S(O)_m-R^{12}$ steht, wobei

$R^{12}$ für gegebenenfalls substituiertes Alkyl mit 1 bis 6 Kohlenstoffatomen oder für gegebenenfalls substituiertes Aryl steht und

m für eine Zahl 0, 1 oder 2 steht,

$R^7$ und $R^8$ unabhängig voneinander für Halogen oder für gegebenenfalls substituiertes Alkyl mit 1 bis 6 Kohlenstoffatomen stehen,

$R^9$ für gegebenenfalls substituiertes Alkyl mit 1 bis 6 Kohlenstoffatomen, für Alkenyl oder Alkinyl mit jeweils 3 bis 6 Kohlenstoffatomen steht oder

$R^6$ und $R^7$ bzw. $R^6$ und $R^8$ gemeinsam mit dem angrenzenden Kohlenstoffatom für einen gegebenenfalls substituierten, gesättigten oder ungesättigten Ring mit 3 bis 8 Ringatomen stehen, der außer Kohlenstoff- auch Sauerstoff- und Schwefelatome als Ringglieder enthalten kann, oder

$R^5$ und $R^7$ bzw. $R^5$ und $R^{10}$ bzw. $R^7$ und $R^9$ bzw. $R^8$ und $R^9$ bzw. $R^7$ und $R^8$ gemeinsam für eine Alkylenkette mit 2 bis 6 Kohlenstoffatomen oder eine Alkenylenkette mit 2 bis 6 Kohlenstoffatomen stehen, welche jeweils durch Halogen, $C_1$-$C_4$-Alkyl oder $C_1$-$C_4$-Alkoxy substituiert sein können, oder

$R^5$ und $R^{11}$ gemeinsam für eine Alkylenkette mit 1 bis 5 Kohlenstoffatomen oder eine Alkenylenkette mit 2 bis 5 Kohlenstoffatomen stehen, welche jeweils durch Halogen, $C_1$-$C_4$-Alkyl oder $C_1$-$C_4$-Alkoxy substituiert sein können, oder

X und $R^9$ gemeinsam für den Rest 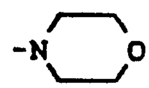 stehen,

gefunden.

Weiterhin wurde gefunden, daß man Pyridylthio-acylanilide der Formel (I) erhält, wenn man

a) Anilin-Derivate der Formel (II),

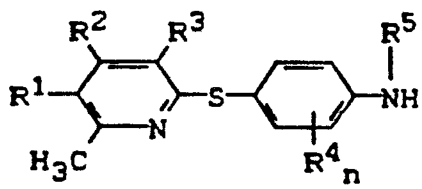

(II)

in welcher

$R^1$, $R^2$, $R^3$, $R^4$, $R^5$ und n die oben angegebene Bedeutung haben,

mit Carbonsäure-Derivaten der Formel (III),

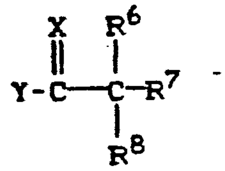

(III)

in welcher

$R^6$, $R^7$, $R^8$ und X die oben angegebene Bedeutung haben und

Y für Hydroxy, Halogen, Acyloxy, Alkoxycarbonyloxy oder Aryloxycarbonyloxy, Alkyl- oder Arylsulfonyloxy,

oder für die Gruppe 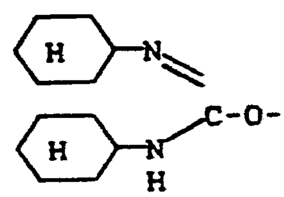

steht,

gegebenenfalls in Gegenwart eines Verdünungsmittels und gegebenenfalls in Gegenwart eines säurebindenden oder wasserbindenden Mittels umsetzt,

oder wenn man

b) Pyridin-Derivate der Formel (IV),

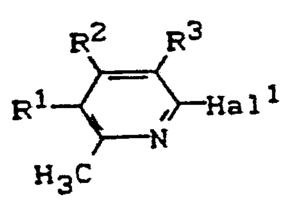

(IV)

in welcher

$R^1$, $R^2$ und $R^3$ die oben angegebene Bedeutung haben und

$Hal^1$ für Halogen steht,

mit Acylanilin-Derivaten der Formel (Va) oder (Vb),

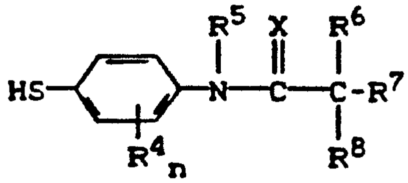

(Va)

oder

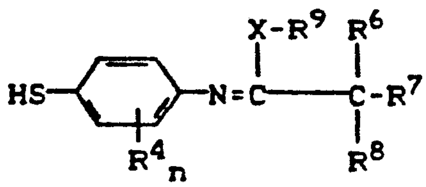

(Vb)

in welcher

$R^4$, $R^5$, $R^6$, $R^7$, $R^8$, $R^9$, X und n die oben angegebene Bedeutung haben,

in Gegenwart eines Verdünungsmittels und gegebenenfalls in Gegenwart eines Säurebindemittels umsetzt,

oder wenn man

c) Carbonsäureanilide der Formel (I), in welcher $R^5$ für Wasserstoff steht und $R^1$, $R^2$, $R^3$, $R^4$, $R^6$, $R^7$, $R^8$, X und n die oben angegebene Bedeutung haben,

mit einem Alkylierungsmittel der Formel (VI)

$$R^5\text{-}Y^1 \qquad (VI),$$

in welcher

$R^5$ die oben angegebene Bedeutung besitzt und

$Y^1$ für Halogen, Alkylsulfonyloxy oder Arylsulfonyloxy steht,

gegebenenfalls in Gegenwart einer Base und gegebenenfalls in Gegenwart eines Verdünnungsmittels umsetzt,

oder wenn man

d) Carbonsäure-imidhalogenide der Formel (VII),

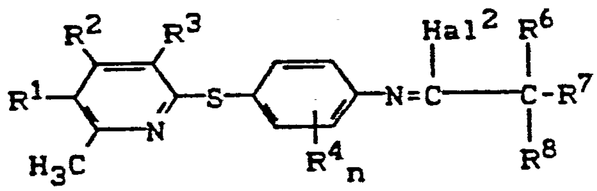

(VII)

in welcher

$R^1$, $R^2$, $R^3$, $R^4$, $R^6$, $R^7$, $R^8$ und n die oben angegebene Bedeutung besitzen und

$Hal^2$ für Chlor oder Brom steht,

mit einem Nucleophil der Formel (VIII),

$$HX\text{-}R^9 \qquad (VIII)$$

in welcher

$R^9$ und X die oben angegebene Bedeutung besitzen,

gegebenenfalls in Gegenwart eines säurebindenden Mittels und gegebenenfalls in Gegenwart eines Lösungsmittels umsetzt,

oder wenn man

e) Carbonsäure-anilide der Formel (Ic),

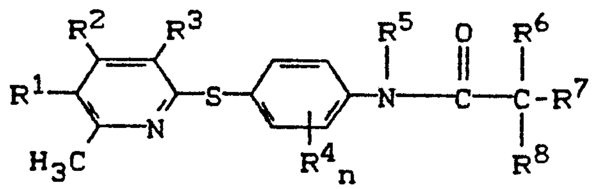

(Ic)

in welcher

$R^1$, $R^2$, $R^3$, $R^4$, $R^5$, $R^6$, $R^7$, $R^8$ und n die oben angegebene Bedeutung besitzen,

mit Schwefelungsreagentien, wie Phosphor-(V)-sulfid oder 2,4-Bis-(4-methoxy-phenyl)-2,4-dithiono-1,3,2,4-dithiaphosphetan (Lawesson-Reagens) gegebenenfalls in Gegenwart eines Verdünnungsmittels umsetzt.

Schließlich wurde gefunden, daß sich die neuen Pyridylthio-acylanilide der Formel (I) durch hervorragende herbizide Wirksamkeit auszeichnen.

Überraschenderweise besitzen die erfindungsgemäßen Pyridylthio-acylanilide der Formel (I) wesentlich bessere herbizide Eigenschaften als die konstitutionell ähnlichsten vorbekannten Stoffe. So lassen sich die erfindungsgemäßen Carbonsäureanilide der Formel (I) wesentlich besser zur Unkrautbekämpfung verwenden als das 2-Phenoxy-4,6-dimethyl-pyrimidin, welches ein strukturell ähnlicher vorbekannter Wirkstoff gleicher Wirkungsart ist.

Im Rahmen der Substituentendefinitionen steht jeweils:

Alkyl für eine geradkettige oder verzweigte Kohlenstoffkette;

Alkoxy für eine geradkettige oder verzweigte Kohlenstoffkette;

Alkylthio für eine geradkettige oder verzweigte Kohlenstoffkette;

Alkenyl für eine geradkettige oder verzweigte Kohlenstoffkette;

Alkinyl für eine geradkettige oder verzweigte Kohlenstoffkette;

Aryl steht für einen aromatischen Kohlenwasserstoffrest mit 6 bis 14 Kohlenstoffatomen, insbesondere mit 6 bis 10 Kohlenstoffatomen.

Aralkyl steht für einen Phenylrest, der über eine Alkylkette mit 1 bis 4 Kohlenstoffatomen gebunden ist;

Halogen steht für Fluor, Chlor, Brom und Iod.

Die erfindungsgemäßen Pyridylthio-acylanilide sind durch die Formel (I) allgemein definiert.

Bevorzugt sind Verbindungen der Formel (I), bei welchen

$R^1$, $R^2$ und $R^3$ unabhängig voneinander für Wasserstoff, Fluor, Chlor, Brom, Cyano, Trifluormethyl, für Alkyl, Alkoxy oder Alkylthio mit jeweils 1 bis 4 Kohlenstoffatomen stehen,

$R^4$ für Fluor, Chlor, Brom, Methyl oder Methoxy steht,

n für eine Zahl 0, 1 oder 2 steht,

Z für die Gruppe (Ia)

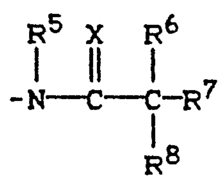

(Ia)

oder die Gruppe (Ib)

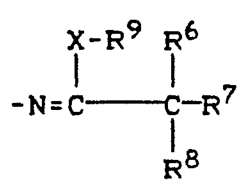

(Ib)

steht, wobei

X für Sauerstoff, Schwefel, eine Gruppe N-$R^{10}$ oder N-O-$R^{11}$ steht, wobei

$R^{10}$ und $R^{11}$ unabhängig voneinander für Wasserstoff oder für Alkyl mit 1 bis 4 Kohlenstoffatomen, für Alkenyl oder Alkinyl mit jeweils 2 bis 4 Kohlenstoffatomen stehen,

$R^5$ für Wasserstoff oder Alkyl mit 1 bis 4 Kohlenstoffatomen, Alkenyl oder Alkinyl mit jeweils 2 bis 6 Kohlenstoffatomen steht,

$R^6$ für Wasserstoff, Fluor, Chlor, Brom, Cyano, für gegebenenfalls einfach oder mehrfach, gleich oder verschieden durch Fluor, Chlor und Brom substituiertes Alkyl mit 1 bis 4 Kohlenstoffatomen, Alkenyl mit 2 bis 4 Kohlenstoffatomen, für jeweils gegebenenfalls einfach bis fünffach, gleich oder verschieden durch Fluor, Chlor, Brom, Trifluormethyl, Methyl und Methoxy substituiertes Phenyl oder Benzyl oder für die Reste -O$R^{12}$ oder -S(O)$_m$-$R^{12}$ steht, wobei

$R^{12}$ für gegebenenfalls einfach oder mehrfach, gleich oder verschieden durch Fluor, Chlor, Brom, Methoxy oder Ethoxy substituiertes Alkyl mit 1 bis 4 Kohlenstoffatomen oder für gegebenenfalls einfach bis fünffach, gleich oder verschieden durch Fluor, Chlor, Brom und $C_1$-$C_4$-Alkyl substituiertes Phenyl steht und

m für eine Zahl 0, 1 oder 2 steht,

$R^7$ und $R^8$ unabhängig voneinander für Fluor, Chlor, Brom oder für gegebenenfalls einfach oder mehrfach, gleich oder verschieden durch Fluor, Chlor und Brom substituiertes Alkyl mit 1 bis 4 Kohlenstoffatomen stehen,

$R^9$ für gegebenenfalls einfach oder mehrfach, gleich oder verschieden durch Halogen oder $C_1$-$C_4$-Alkoxy substituiertes Alkyl mit 1 bis 4 Kohlenstoffatomen, für Alkenyl oder Alkinyl mit jeweils 3 oder 4 Kohlenstoffatomen steht oder

$R^6$ und $R^7$ bzw. $R^6$ und $R^8$ gemeinsam mit dem angrenzenden Kohlenstoffatom für einen gesättigten oder ungesättigten Ring mit 3 bis 7 Ringatomen stehen, der außer Kohlenstoff auch Sauerstoff oder Schwefel enthalten kann, und welcher einfach oder mehrfach, gleich oder verschieden durch Fluor, Chlor oder $C_1$-$C_4$-Alkyl substituiert sein kann, oder

$R^5$ und $R^7$ bzw. $R^5$ und $R^{10}$ bzw. $R^7$ und $R^9$ bzw. $R^8$ und $R^9$ bzw. $R^7$ und $R^8$ gemeinsam für eine Alkylenkette mit 2 bis 5 Kohlenstoffatomen oder eine Alkenylenkette mit 2 bis 5 Kohlenstoffatomen stehen, welche jeweils einfach oder mehrfach, gleich oder verschieden durch Halogen, $C_1$-$C_4$-Alkyl oder $C_1$-$C_4$-Alkoxy substituiert sein können, oder

$R^5$ und $R^{11}$ gemeinsam für eine Alkylenkette mit 1 bis 4 Kohlenstoffatomen oder eine Alkenylenkette mit 2 bis 4 Kohlenstoffatomen stehen, welche jeweils einfach

oder mehrfach, gleich oder verschieden durch Halogen oder $C_1$-$C_4$-Alkyl substituiert sein können, oder

X und $R^9$ gemeinsam für den Rest 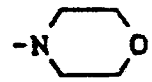 stehen.

Besonders bevorzugt sind Verbindungen der Formel (I), bei welchen

$R^1$, $R^2$ und $R^3$ unabhängig voneinander für Wasserstoff, Chlor, Cyano, Trifluormethyl, Methyl, Ethyl oder Methoxy stehen,

$R^4$ für Fluor, Chlor, Methyl oder Methoxy steht,

n für eine Zahl 0 oder 1 steht,

Z für die Gruppe (Ia)

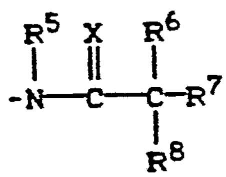

(Ia)

oder die Gruppe (Ib)

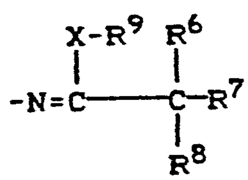

(Ib)

steht, wobei

X für Sauerstoff, Schwefel, eine Gruppe $N-R^{10}$ oder $N-O-R^{11}$ steht, wobei

$R^{10}$ und $R^{11}$ unabhängig voneinander für Wasserstoff oder für Alkyl mit 1 bis 3 Kohlenstoffatomen oder für Alkenyl oder Alkinyl mit jeweils 2 oder 3 Kohlenstoffatomen stehen,

$R^5$ für Wasserstoff oder für Alkyl mit 1 bis 3 Kohlenstoffatomen, Alkenyl mit 2 bis 4 Kohlenstoffatomen, oder Alkinyl mit 3 bis 4 Kohlenstoffatomen steht,

$R^6$ für Wasserstoff, Fluor, Chlor, Brom, Cyano, für gegebenenfalls einfach bis fünffach, gleich oder verschieden durch Fluor und Chlor substituiertes Alkyl mit 1 bis 4 Kohlenstoffatomen, für jeweils gegebenenfalls einfach bis dreifach, gleich oder verschieden durch Fluor, Chlor, Trifluormethyl und Methyl substituiertes Phenyl oder Benzyl oder für die Reste $-OR^{12}$ oder $-S(O)_m-R^{12}$ steht, wobei

$R^{12}$ für gegebenenfalls einfach bis fünffach, gleich oder verschieden durch Fluor und Chlor substituiertes Alkyl mit 1 bis 4 Kohlenstoffatomen oder für gegebenenfalls einfach bis dreifach, gleich oder verschieden durch Fluor, Chlor und $C_1$-$C_2$-Alkyl substituiertes Phenyl steht und

m für eine Zahl 0, 1 oder 2 steht,

$R^7$ und $R^8$ unabhängig voneinander für Fluor, Chlor oder für gegebenenfalls einfach bis fünffach, gleich oder verschieden durch Fluor und Chlor substituiertes Alkyl mit 1 bis 2 Kohlenstoffatomen stehen,

$R^9$ für gegebenenfalls einfach bis fünffach, gleich oder verschieden durch Halogen oder $C_1$- $C_4$-Alkoxy substituiertes Alkyl mit 1 oder 2 Kohlenstoffatomen oder für Alkenyl mit 3 oder 4 Kohlenstoffatomen steht oder

$R^6$ und $R^7$ bzw. $R^6$ und $R^8$ gemeinsam mit dem angrenzenden Kohlenstoffatom für einen gesättigten oder ungesättigten Ring mit 3 bis 6 Ringatomen stehen, der außer Kohlenstoff auch Sauerstoff und Schwefel enthalten kann, und welcher gegebenenfalls einfach bis dreifach, gleich oder verschieden durch Fluor, Chlor, Methyl, Ethyl, n-Propyl und i-Propyl substituiert sein kann, oder

$R^7$ und $R^8$ gemeinsam für eine Alkylenkette mit 2 bis 5 Kohlenstoffatomen stehen, welche einfach bis fünffach, gleich oder verschieden durch Fluor, Chlor, Methyl, Ethyl oder Methoxy substituiert sein kann, oder

$R^5$ und $R^7$ bzw. $R^5$ und $R^{10}$ bzw. $R^7$ und $R^9$ bzw. $R^8$ und $R^9$ gemeinsam für eine Alkylenkette mit 2 bis 3 Kohlenstoffatomen oder eine Alkenylenkette mit 3 bis 4 Kohlenstoffatomen stehen, welche jeweils einfach bis dreifach, gleich oder verschieden durch Fluor, Chlor, Methyl, Ethyl und Methoxy substituiert sein können, oder

$R^5$ und $R^{11}$ gemeinsam für eine Alkylenkette mit 1 oder 2 Kohlenstoffatomen stehen, welche einfach bis dreifach, gleich oder verschieden durch Methyl oder Ethyl substituiert sein kann.

Eine Gruppe ganz besonders bevorzugter Pyridylthio-acylanilide der Formel (I) sind diejenigen, in welchen X für Sauerstoff steht und die anderen Substituenten die oben als besonders bevorzugt angegebene Bedeutung haben.

Eine andere ganz besonders bevorzugte Gruppe sind diejenigen Verbindungen der Formel (I), in denen X für Schwefel steht und die anderen Substituenten die oben als besonders bevorzugt angegebene Bedeutung haben.

Ganz besonders bevorzugt sind auch solche Verbindungen, in denen X für N-$R^{10}$ oder N-O$R^{11}$ steht und die restlichen Substituenten die oben als besonders bevorzugt angegebene Bedeutung haben.

Beispielhaft seien die in der folgenden Tabelle 1 aufgeführten Pyridylthio-acylanilide der Formel (I) genannt:

Tabelle 1

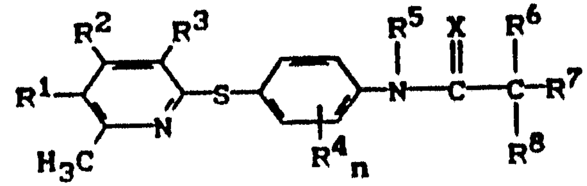

(Ia)

| $R^1$ | $R^2$ | $R^3$ | $R^4$ | n | $R^5$ | X | $R^6$ | $R^7$ | $R^8$ |
|---|---|---|---|---|---|---|---|---|---|
| H | H | H | - | 0 | H | O | Cl | Cl | Cl |
| H | H | H | - | 0 | H | O | H | $CH_3$ | $CH_3$ |
| H | H | H | - | 0 | H | O | H | $C_2H_5$ | $C_2H_5$ |
| H | H | H | - | 0 | H | O | $CH_2F$ | $CH_3$ | $CH_3$ |
| H | H | H | - | 0 | H | O | $C_2H_5$ | $CH_3$ | $CH_3$ |
| H | H | H | - | 0 | H | O | $C_3H_7$ | $CH_3$ | $CH_3$ |
| H | H | H | - | 0 | H | O | $OCH_3$ | $CH_3$ | $CH_3$ |
| H | H | H | - | 0 | H | O | $CH_2OCH_3$ | $CH_3$ | $CH_3$ |
| H | H | H | - | 0 | H | O | 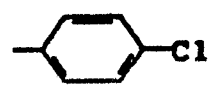 | $CH_3$ | $CH_3$ |

<u>Tabelle 1</u> (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | H | H | - | 0 | H | O | 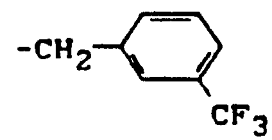 | $CH_3$ | $CH_3$ |
| H | H | H | - | 0 | H | O | CN | $CH_3$ | $CH_3$ |
| H | H | H | - | 0 | H | O | $CH_3$ | | $-CH_2-CH_2-$ |
| H | H | H | - | 0 | H | O | $CH_3$ | | $-(CH_2)_5-$ |
| H | H | H | - | 0 | H | O | | $-CH_2-O-CH_2-$ | $CH_3$ |
| H | H | H | - | 0 | H | O | | $-O-(CH_2)_4-$ | $CH_3$ |
| H | H | H | - | 0 | H | S | $CH_3$ | $CH_3$ | $CH_3$ |
| H | H | H | - | 0 | $CH_3$ | O | $CH_3$ | $CH_3$ | $CH_3$ |
| H | H | H | - | 0 | $CH_2-CH=CH_2$ | O | $CH_3$ | $CH_3$ | $CH_3$ |
| H | H | H | - | 0 | $CH_2-C{\equiv}CH$ | O | $CH_3$ | $CH_3$ | $CH_3$ |
| H | H | H | 2-F | 1 | H | O | $CH_3$ | $CH_3$ | $CH_3$ |
| H | H | H | 3-F | 1 | H | O | $CH_3$ | $CH_3$ | $CH_3$ |

Tabelle 1 (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | H | H | 2-Cl | 1 | H | O | $CH_3$ | $CH_3$ | $CH_3$ |
| H | H | H | 3-Cl | 1 | H | O | $CH_3$ | $CH_3$ | $CH_3$ |
| H | H | H | 2-Br | 1 | H | O | $CH_3$ | $CH_3$ | $CH_3$ |
| H | H | H | 2-$CH_3$ | 1 | H | O | $CH_3$ | $CH_3$ | $CH_3$ |
| H | H | H | 3-$CH_3$ | 1 | H | O | $CH_3$ | $CH_3$ | $CH_3$ |
| H | H | H | 2-$OCH_3$ | 1 | H | O | $CH_3$ | $CH_3$ | $CH_3$ |
| H | H | H | 3-$OCH_3$ | 1 | H | O | $CH_3$ | $CH_3$ | $CH_3$ |
| H | H | Cl | - | 0 | H | O | $CH_3$ | $CH_3$ | $CH_3$ |
| H | H | F | - | 0 | H | O | $CH_3$ | $CH_3$ | $CH_3$ |
| H | H | CN | - | 0 | H | O | $CH_3$ | $CH_3$ | $CH_3$ |
| H | H | $CH_3$ | - | 0 | H | O | $CH_3$ | $CH_3$ | $CH_3$ |
| H | Cl | H | - | 0 | H | O | $CH_3$ | $CH_3$ | $CH_3$ |

0243868

Tabelle 1 (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | F | H | - | 0 | H | O | $CH_3$ | $CH_3$ | $CH_3$ |
| H | $OCH_3$ | H | - | 0 | H | O | $CH_3$ | $CH_3$ | $CH_3$ |
| H | $SCH_3$ | H | - | 0 | H | O | $CH_3$ | $CH_3$ | $CH_3$ |
| H | $CH_3$ | H | - | 0 | H | O | Cl | Cl | Cl |
| H | $CH_3$ | H | - | 0 | H | O | H | $CH_3$ | $CH_3$ |
| H | $CH_3$ | H | - | 0 | H | O | $CH_2F$ | $CH_3$ | $CH_3$ |
| H | $CH_3$ | H | - | 0 | H | O | $-CH=CH_2$ | $CH_3$ | $CH_3$ |
| H | $CH_3$ | H | - | 0 | H | O | $OCH_3$ | $CH_3$ | $CH_3$ |
| H | $CH_3$ | H | - | 0 | H | O | $-CH_2OCH_3$ | $CH_3$ | $CH_3$ |
| H | $CH_3$ | H | - | 0 | H | O | $SCH_3$ | $CH_3$ | $CH_3$ |
| H | $CH_3$ | H | - | 0 | H | O | $-SO_2CH_3$ | $CH_3$ | $CH_3$ |
| H | $CH_3$ | H | - | 0 | H | O | 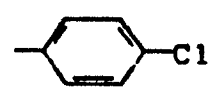 | $CH_3$ | $CH_3$ |

Tabelle 1 (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | $CH_3$ | H | - | 0 | H | O | 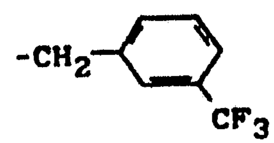 | $CH_3$ | $CH_3$ |
| H | $CH_3$ | H | - | 0 | H | O | $CH_3$ | | $-CH_2-CH_2-$ |
| H | $CH_3$ | H | - | 0 | H | O | $CH_3$ | | $-(CH_2)_4-$ |
| H | $CH_3$ | H | - | 0 | H | O | | $-CH_2-O-CH_2-$ | $CH_3$ |
| H | $CH_3$ | H | - | 0 | H | S | $CH_3$ | $CH_3$ | $CH_3$ |
| H | $CH_3$ | H | - | 0 | $CH_3$ | O | $CH_3$ | $CH_3$ | $CH_3$ |
| H | $CH_3$ | H | - | 0 | $-CH_2-CH=CH_2$ | O | $CH_3$ | $CH_3$ | $CH_3$ |
| H | $CH_3$ | H | - | 0 | $-CH_2-C\equiv CH$ | O | $CH_3$ | $CH_3$ | $CH_3$ |
| H | $CH_3$ | H | - | 0 | $-CH_2-$ | O | $CH_3$ | $-CH_2-$ | $CH_3$ |
| H | $CH_3$ | H | - | 0 | $-CH_2-$ | O | $CH_3$ | $-CH_2-CH_2-$ | $CH_3$ |
| H | $CH_3$ | H | - | 0 | $(CH_2)_2$ —— | N | $CH_3$ | $CH_3$ | $CH_3$ |
| H | $CH_3$ | H | - | 0 | $(CH_2)_3$ —— | N | $CH_3$ | $CH_3$ | $CH_3$ |

**Tabelle 1** (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | $CH_3$ | H | 2-F | 1 | H | O | $CH_3$ | $CH_3$ | $CH_3$ |
| H | $CH_3$ | H | 3-F | 1 | H | O | $CH_3$ | $CH_3$ | $CH_3$ |
| H | $CH_3$ | H | 2-Cl | 1 | H | O | $CH_3$ | $CH_3$ | $CH_3$ |
| H | $CH_3$ | H | 3-Cl | 1 | H | O | $CH_3$ | $CH_3$ | $CH_3$ |
| H | $CH_3$ | H | 3,5-Cl | 2 | H | O | $CH_3$ | $CH_3$ | $CH_3$ |
| H | $CH_3$ | H | 2-Br | 1 | H | O | $CH_3$ | $CH_3$ | $CH_3$ |
| H | $CH_3$ | H | 2-$CH_3$ | 1 | H | O | $CH_3$ | $CH_3$ | $CH_3$ |
| H | $CH_3$ | H | 3-$CH_3$ | 1 | H | O | $CH_3$ | $CH_3$ | $CH_3$ |
| H | $CH_3$ | H | 3-$OCH_3$ | 1 | H | O | $CH_3$ | $CH_3$ | $CH_3$ |
| H | $CH_3$ | H | 3-$OCH_3$ | 1 | H | O | $CH_3$ | $CH_3$ | $CH_3$ |
| H | $CH_3$ | F | - | 0 | H | O | $CH_3$ | $CH_3$ | $CH_3$ |
| H | $CH_3$ | Cl | - | 0 | H | O | $CH_3$ | $CH_3$ | $CH_3$ |

Tabelle 1 (Fortsetzung)

| $R^1$ | $R^2$ | $R^3$ | $R^4$ | n | $R^5$ | X | $R^6$ | $R^7$ | $R^8$ |
|---|---|---|---|---|---|---|---|---|---|
| H | $CH_3$ | $CH_3$ | - | 0 | H | O | $CH_3$ | $CH_3$ | $CH_3$ |
| H | $CF_3$ | H | - | 0 | H | O | $CH_3$ | $CH_3$ | $CH_3$ |
| H | $C_2H_5$ | H | - | 0 | H | O | $CH_3$ | $CH_3$ | $CH_3$ |
| H | $C_3H_7$ | H | - | 0 | H | O | $CH_3$ | $CH_3$ | $CH_3$ |
| H | $CH(CH_3)_2$ | H | - | 0 | H | O | $CH_3$ | $CH_3$ | $CH_3$ |
| F | H | H | - | 0 | H | O | $CH_3$ | $CH_3$ | $CH_3$ |
| Cl | $CH_3$ | H | - | 0 | H | O | $CH_3$ | $CH_3$ | $CH_3$ |
| $CH_3$ | $CH_3$ | H | - | 0 | H | O | $CH_3$ | $CH_3$ | $CH_3$ |

**Tabelle 2**

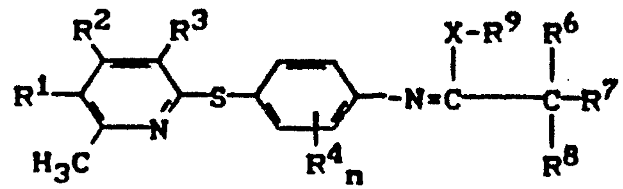

(Ib)

| $R^1$ | $R^2$ | $R^3$ | $R^4$ | n | X | $R^6$ | $R^7$ | $R^8$ | $R^9$ |
|---|---|---|---|---|---|---|---|---|---|
| H | H | H | - | 0 | O | $CH_3$ | $CH_3$ | $CH_3$ | $CH_3$ |
| H | H | H | - | 0 | O | $CH_3$ | $CH_3$ | $-CH_2-CH_2$ | |
| H | H | H | - | 0 | O | $CH_3$ | $CH_3$ | $-(CH_2)_3-$ | |
| H | H | $CH_3$ | - | 0 | S | $CH_3$ | $CH_3$ | $CH_3$ | $CH_3$ |
| H | H | $CH_3$ | - | 0 | S | $CH_3$ | $CH_3$ | $CH_3$ | $-CH_2-CH=CH_2$ |
| H | H | $CH_3$ | - | 0 | S | $CH_3$ | $CH_3$ | $CH_3$ | $-CH_2-C\equiv CH$ |
| H | H | $CH_3$ | - | 0 | S | $CH_3$ | $CH_3$ | $-CH_2-CH_2-$ | |
| H | H | $CH_3$ | - | 0 | $CH_3-N$ | $CH_3$ | $CH_3$ | $CH_3$ | $CH_3$ |
| H | H | $CH_3$ | - | 0 | $CH_3-N$ | $CH_3$ | $CH_3$ | $CH_3$ | $CH_2-CH=CH_2$ |
| H | H | $CH_3$ | - | 0 | $CH_2-CH_2-N$ | $CH_3$ | $CH_3$ | $CH_3$ | $O-CH_2-CH_2$ |

Verwendet man 4-(4,6-Dimethyl-pyridyl-2-thio)-anilin und Pivalsäurechlorid als Ausgangsstoffe, so kann der Verlauf des erfindungsgemäßen Verfahrens (a) durch das folgende Formelschema wiedergegeben werden:

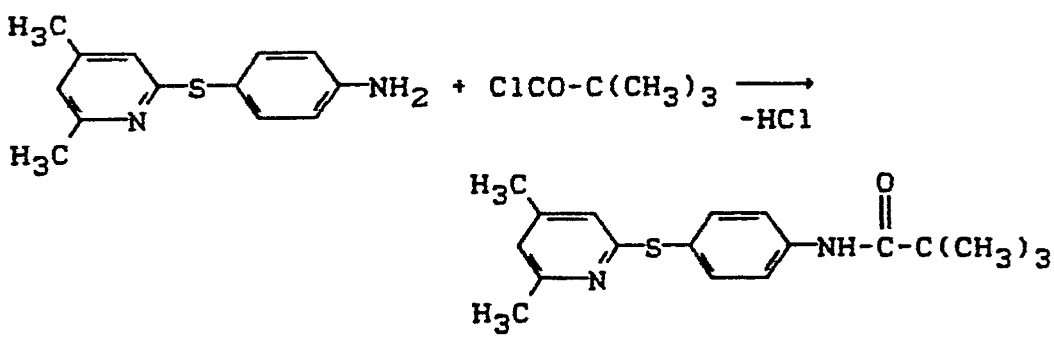


Verwendet man 4-(6-Methyl-pyridyl-2-thio)-anilin und Isobuttersäureanhydrid als Ausgangsstoffe, so kann der Verlauf des erfindungsgemäßen Verfahrens (a) durch das folgende Formelschema wiedergegeben werden:

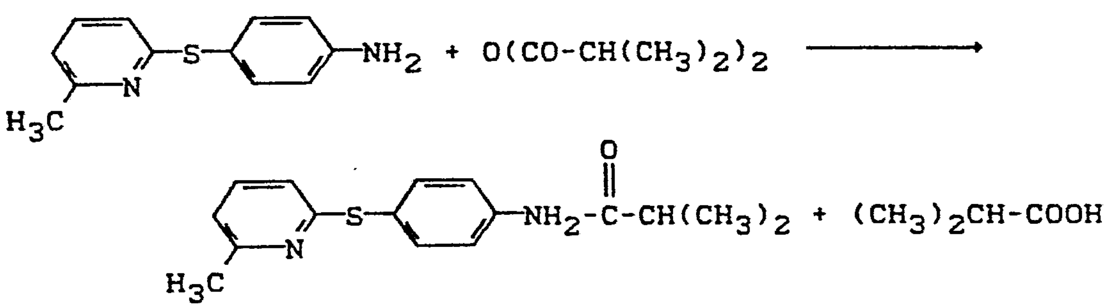

Verwendet man 4-(5,6-Dimethyl-pyridyl-2-thio)-anilin und α-Methoxy-isobuttersäure-kohlensäure-ethylester-anhydrid als Ausgangsstoffe, so kann der Verlauf des erfindungsgemäßen Verfahrens (a) durch das folgende Formelschema wiedergegeben werden:

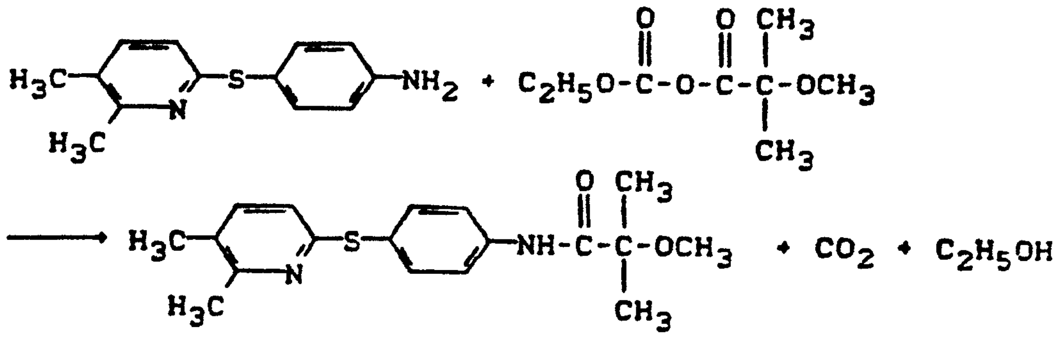

Verwendet man 4-(4,5,6-Trimethyl-pyridyl-2-thio)-anilin und O-Pivaloyloxy-dicyclohexyl-isoharnstoff als Ausgangsstoffe, so kann der Verlauf des erfindungsgemäßen Verfahrens (a) durch das folgende Formelschema wiedergegeben werden:

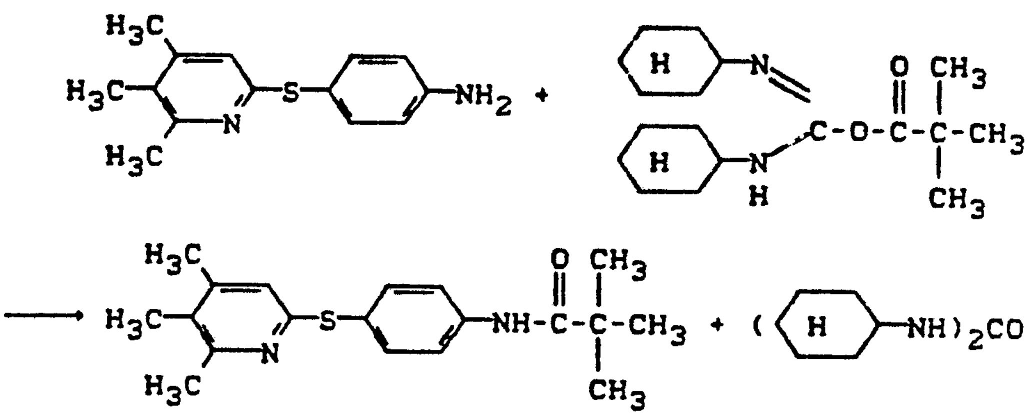

Verwendet man 2-Chlor-4,6-dimethyl-pyridin und 4-Pivaloylaminothiophenol als Ausgangsstoffe, so kann der Verlauf des erfindungsgemäßen Verfahrens (b) durch das folgende Formelschema wiedergegeben werden:

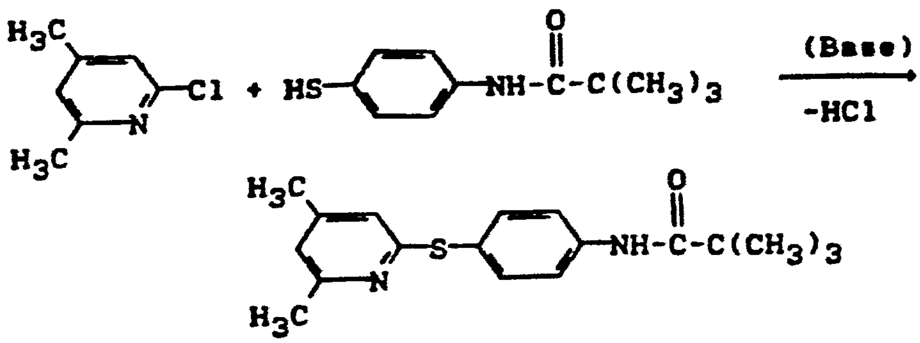


Verwendet man Pivalsäure-4-(4,6-dimethyl-pyridyl-2-thio)-anilid und Allylbromid als Ausgangsstoffe, so kann der Verlauf des erfindungsgemäßen Verfahrens (c) durch das folgende Formelschema wiedergegeben werden:

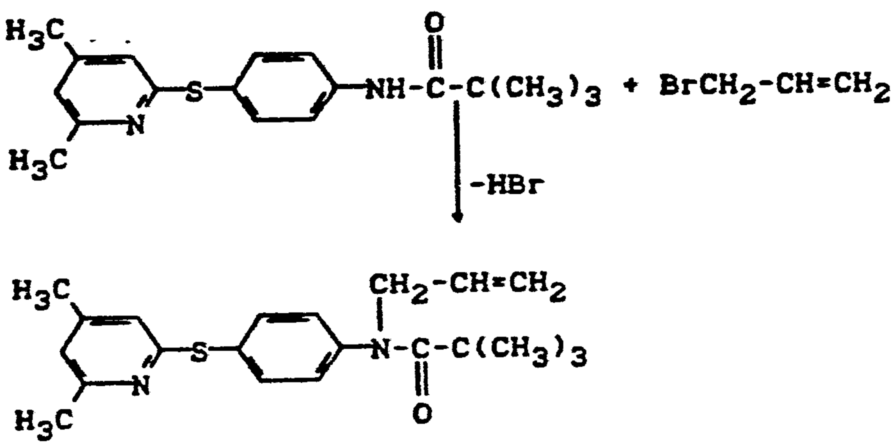


Verwendet man Thiopivalsäure-4-(4,6-dimethyl-pyridyl-2-thio)-anilid und Methyliodid als Ausgangsstoffe, so kann der Verlauf des erfindungsgemäßen Verfahrens (c) durch das folgende Formelschema wiedergegeben werden:

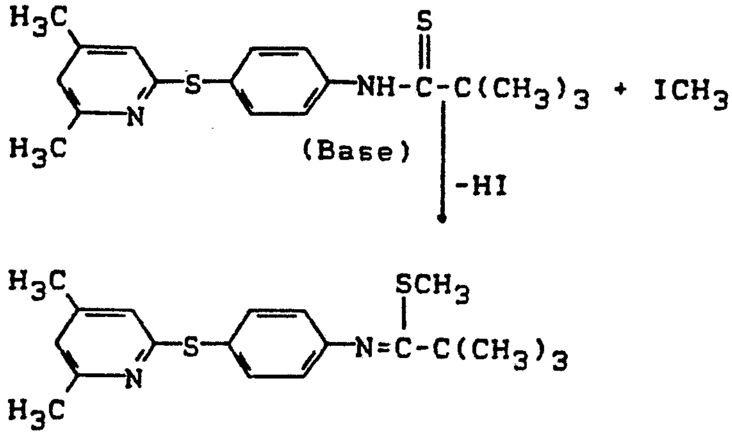


Verwendet man Pivaloyl-4-(4,6-dimethyl-pyridyl-2-thio)-phenylimidchlorid und Methanol bzw. Methanthiol bzw. Dimethylamin als Ausgangsstoffe, so kann der Verlauf des erfindungsgemäßen Verfahrens (d) durch das folgende Formelschema wiedergegeben werden:

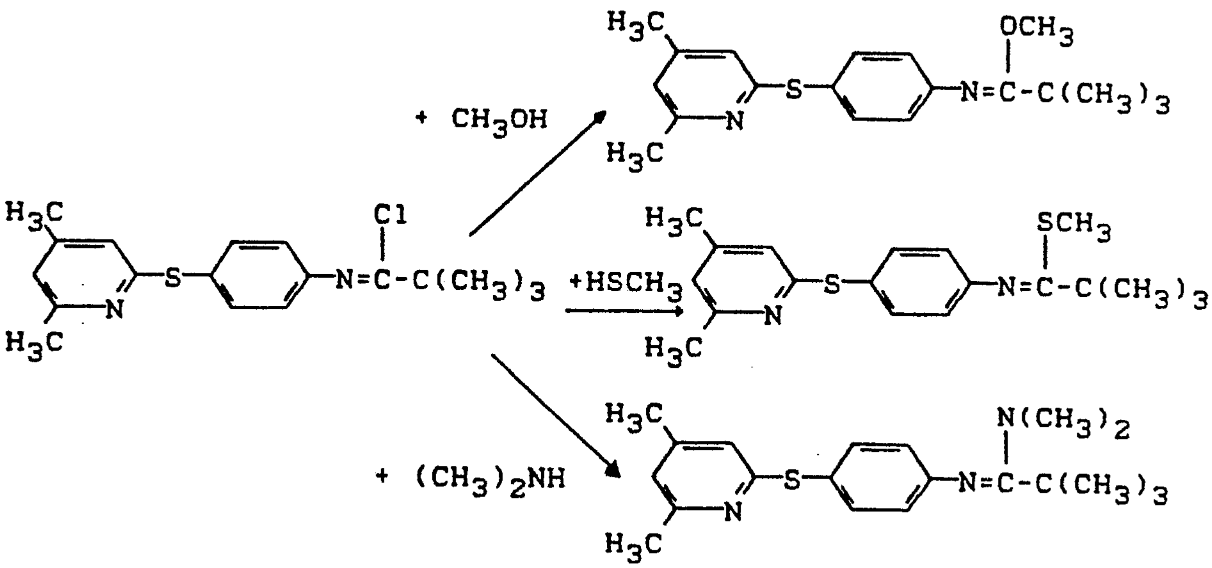

Verwendet man Pivalsäure-4-(4,6-dimethyl-pyridyl-2-thio)-anilid und Phosphor-V-sulfid als Ausgangsstoffe, so kann der Verlauf des erfindungsgemäßen Verfahrens (e) durch das folgende Formelschema wiedergegeben werden:

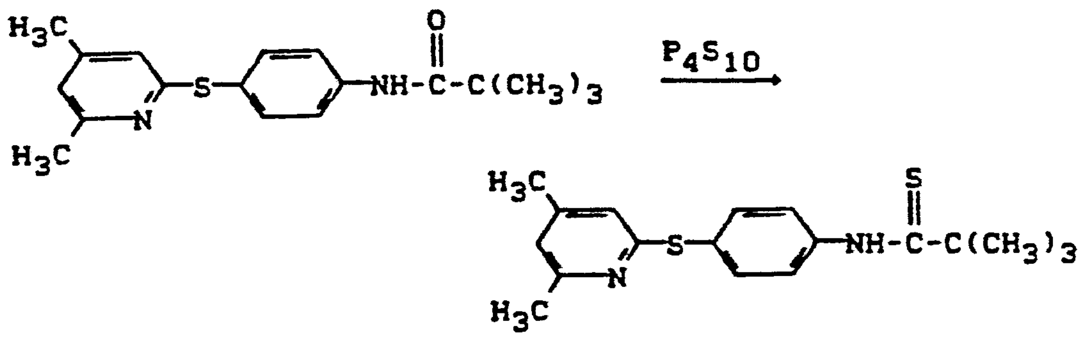


Die bei dem erfindungsgemäßen Verfahren (a) als Ausgangsstoffe benötigten Anilin-Derivate sind durch die Formel (II) allgemein definiert. In dieser Formel haben $R^1$, $R^2$, $R^3$, $R^4$, $R^5$ und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Anilin-Derivate der Formel (II) sind bisher nicht bekannt. Sie lassen sich herstellen, indem man

(A) Pyridin-Derivate der Formel (IV),

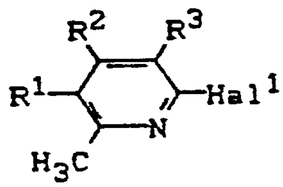

(IV)

in welcher

$R^1$, $R^2$, $R^3$ und $Hal^1$ die oben angegebene Bedeutung haben,

mit 4-Amino-thiophenolen der Formel (IX),

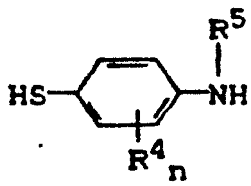

(IX)

in welcher

$R^4$, $R^5$ und n die oben angegebene Bedeutung haben,

in Gegenwart eines Säurebindemittels sowie gegebenenfalls in Gegenwart eines Verdünnungsmittels umsetzt,

oder

(B) 2-(4-Nitro-phenylthio)-pyridin-Derivate der Formel (X),

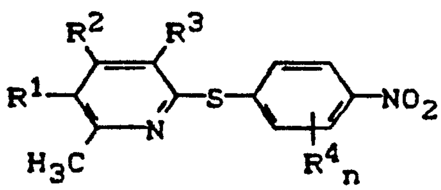

(X)

in welcher

$R^1$, $R^2$, $R^3$, $R^4$ und n die oben angegebene Bedeutung haben,

gegebenenfalls in Gegenwart eines Verdünnungsmittels nach üblichen Methoden reduziert.

Die bei dem obigen Verfahren (A) als Ausgangsstoffe benötigten Pyridin-Derivate sind durch die Formel (IV) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. $Hal^1$ steht vorzugsweise für Fluor, Chlor oder Brom.

Als Beispiele für Pyridin-Derivate der Formel (IV) seien genannt:

2-Chlor-6-methyl-pyridin
2-Brom-6-methyl-pyridin
2,3-Dichlor-6-methyl-pyridin
2,4-Dichlor-6-methyl-pyridin
2-Chlor-5-fluor-6-methyl-pyridin
2-Chlor-4-methoxy-6-methyl-pyridin
2-Chlor-4-methylmercapto-6-methyl-pyridin
2-Chlor-3-cyan-6-methyl-pyridin
2-Chlor-3,6-dimethyl-pyridin
2-Chlor-4,6-dimethyl-pyridin
2-Fluor-4,6-dimethyl-pyridin
2-Chlor-6-methyl-4-propyl-pyridin
2-Chlor-6-methyl-4-isopropyl-pyridin
2-Chlor-6-methyl-4-trifluormethyl-pyridin

2,3-Dichlor-4,6-dimethyl-pyridin
2-Chlor-5-fluor-4,6-dimethyl-pyridin
2-Chlor-3-cyan-4,6-dimethyl-pyridin
2-Chlor-3,4,6-trimethyl-pyridin
2-Chlor-4,5,6-trimethyl-pyridin

Die Pyridin-Derivate der Formel (IV) sind bekannt oder lassen sich nach im Prinzip bekannten Methoden in einfacher Weise herstellen. So erhält man Pyridin-Derivate der Formel (IV) z.B. dadurch, daß man 2-Hydroxy-pyridin-Derivate (Dihydro-pyridon-2-Derivate) mit anorganischen Säurehalogeniden, wie z.B. Phosphoroxychlorid oder Phosphorpentachlorid, umsetzt, oder auch dadurch, daß man entsprechende 2-Amino-pyridin-Derivate mit salpetriger Säure in Gegenwart von Halogenwasserstoffsäuren umsetzt.

Die bei dem Verfahren (A) weiterhin als Ausgangsstoffe benötigten 4-Amino-thiophenole sind durch die Formel (IX) definiert. In dieser Formel haben $R^4$, $R^5$ und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Als Beispiele für 4-Amino-thiophenole der Formel (IX) seien genannt:

4-Amino-thiophenol
4-Methylamino-thiophenol
2-Fluor-4-amino-thiophenol
3-Fluor-4-amino-thiophenol

2-Chlor-4-amino-thiophenol
3-Chlor-4-amino-thiophenol
2,6-Dichlor-4-amino-thiophenol
2-Methyl-4-amino-thiophenol
3-Methyl-4-amino-thiophenol
2-Methoxy-4-amino-thiophenol
3-Methoxy-4-amino-thiophenol

Die 4-Amino-thiophenole der Formel (IX) sind bekannt oder lassen sich nach im Prinzip bekannten Methoden in einfacher Weise herstellen.

Als Säurebindemittel können bei der Durchführung des Verfahrens (A) alle üblicherweise für derartige Umsetzungen verwendbaren Säureakzeptoren verwendet werden. Vorzugsweise verwendbar sind Alkali- und Erdalkali-oxide, -hydroxide und -carbonate, wie Natriumhydroxid, Kaliumhydroxid, Calciumhydroxid, Natriumcarbonat und Kaliumcarbonat, ferner Alkali-alkoholate, -amide und -hydride, wie z.B. Natriummethylat, Natriumethylat, Kalium-tert.-butylat, Natriumamid und Natriumhydrid.

Als Verdünnungsmittel können bei der Durchführung des Verfahrens (A) alle üblichen inerten oganischen Solventien verwendet werden. Vorzugsweise infrage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan und Dimethylformamid.

Die Reaktionstemperaturen können bei der Durchführung des Verfahrens (A) innerhalb eines größeren Bereiches variiert werden. Im allgemeinen arbeitet man bei Temperaturen zwischen 0° C und 200° C, vorzugsweise zwischen 50° C und 150° C.

Die Umsetzung nach dem Verfahren (A) wird im allgemeinen unter Normaldruck vorgenommen.

Bei der Durchführung des Verfahrens (A) setzt man die Ausgangsstoffe der Formeln (IV) und (IX) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (B) als Ausgangsstoffe benötigten 2-(4-Nitro-phenylthio)-pyridin-Derivate sind durch die Formel (X) definiert. In dieser Formel haben $R^1$, $R^2$, $R^3$, $R^4$ und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Die Verbindungen der Formel (X) sind bekannt oder lassen sich nach im Prinzip bekannten Methoden in einfacher Weise herstellen. So erhält man Verbindungen der Formel (X) z.B. dadurch, daß man Pyridin-Derivate der Formel (IV),

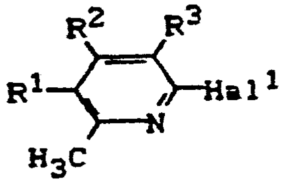

(IV)

in welcher

$R^1$, $R^2$, $R^3$ und $Hal^1$ die oben angegebene Bedeutung haben,

mit 4-Nitro-thiophenolen der Formel (XI),

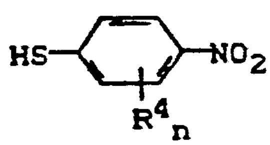

(XI)

in welcher

$R^4$ und n die oben angegebene Bedeutung haben,

in Gegenwart eines Säurebindemittels und gegebenenfalls in Gegenwart eines Verdünnungsmittels bei Temperaturen zwischen 0° C und 200° C, vorzugsweise zwischen 50° C und 150° C, umsetzt. Als Säurebindemittel und Verdünnungsmittel kommen hierbei vorzugsweise diejenigen Stoffe in Betracht, die bereits im Zusammenhang mit dem Verfahren (A) als vorzugsweise verwendbare Säureakzeptoren und Solventien genannt wurden.

Nach einem weiteren Verfahren gewinnt man die als Zwischenprodukte benötigten Verbindungen der Formel (X) dadurch, daß man 2-Mercapto-pyridine der Formel (XII)

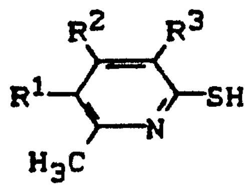

(XII)

in welcher $R^1$, $R^2$ und $R^3$ die oben angegebene Bedeutung besitzen, mit 4-Halogen-nitro-benzolen der Formel (XIII),

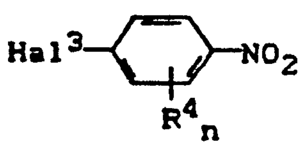

(XIII)

in der $R^4$ und n die oben angegebene Bedeutung besitzen und $Hal^3$ für Fluor, Chlor oder Brom steht, in Gegenwart eines Säurebindemittels und gegebenenfalls in Gegenwart eines Verdünnungsmittels bei Temperaturen zwischen 0°C und 200°, vorzugsweise zwischen 50°C und 150°C umsetzt. Als Säurebindemittel und Verdünnungsmittel kommen hierbei vorzugsweise diejenigen Stoffe in Betracht, die bereits im Zusammenhang mit dem Verfahren (A) in entsprechender Weise genannt wurden.

Als Reduktionsmittel kommen bei dem Verfahren (B) alle diejenigen Stoffe infrage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen-(II)- und Zinn-(II)-Salze, und außerdem Nichtmetall-Verbindungen in niederen Wertigkeitsstufen, wie z.B. Salze des Schwefelwasserstoffes, Alkalisulfite und Alkalidithionite. Im übrigen kann die Reduktion auch durch katalytische Hydrierung mit Wasserstoff in Gegenwart eines Katalysators, wie z.B. Raney-Nickel, erfolgen.

Als Verdünnungsmittel kommen bei dem Verfahren (B) alle übliche für derartige Reduktionen geeigneten organischen Solventien in Betracht. Die Reaktionstemperaturen können innerhalb eines größeren Bereiches variiert werden. Sie entsprechen den Temperaturen, die bei analogen Reaktionen angewandt werden.

Die Durchführung der Reduktion nach dem Verfahren (B) und die Aufarbeitung des anfallenden Reaktionsgemisches erfolgen nach üblichen Methoden.

Die bei dem erfindungsgemäßen Verfahren (a) als Reaktionskomponenten weiterhin benötigten Carbonsäuren und deren Derivate sind durch die Formel (III) eindeutig definiert. In dieser Formel haben $R^6$, $R^7$, $R^8$ und X vorzugsweise diejenigen Bedeutungen, die bereits im Zusammenhang mit der Beschreibung der erfindungsgemäßen Stoffe der Formel (I) vorzugsweise für diese Reste genannt wurden. Y steht

vorzugsweise für Hydroxy, Fluor, Chlor, Brom, Acyloxy, $C_1$-$C_4$-Alkoxycarbonyloxy, Phenyl- oder p-Tolyloxycarbonyloxy, Methyl- oder Ethylsulfonyloxy oder Phenyl- oder p-Tolylsulfonyloxy oder für die Gruppe

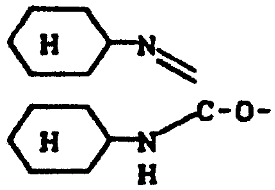

Als Beispiele für Verbindungen der Formel (III) seien genannt:

Trichloressigsäure
Trifluoressigsäure
α,α-Dichlorpropionsäure
Isobuttersäure
α-Chlor-isobuttersäure
α-Brom-isobuttersäure
α-Methoxy-isobuttersäure
α-Phenoxy-isobuttersäure
α-(4-Chlor-phenoxy)-isobuttersäure
α-(2-Methyl-4-chlor-phenoxy)-isobuttersäure
α-Methylmercapto-isobuttersäure
α-Methylsulfonyl-isobuttersäure
α-Methyl-buttersäure
Pivalinsäure
β-Fluor-pivalinsäure
β-Chlor-pivalinsäure

β,β'-Difluor-pivalinsäure
β,β'-Dichlor-pivalinsäure
β,β',β"-Trifluor-pivalinsäure
β,β',β"-Trichlor-pivalinsäure
α,α-Dimethyl-buttersäure
α-Vinyl-isobuttersäure
α-Ethinyl-isobuttersäure
α,α-Dimethyl-valeriansäure
α-Methyl-α-ethyl-buttersäure
α,α-Dimethyl-phenylessigsäure
α,α-Dimethyl-(4-chlor-phenyl)-essigsäure
α,α-Dimethyl-(3,4-dichlor-phenyl)-essigsäure
α,α-Dimethyl-(3-trifluormethyl-phenyl)-essigsäure
α-Benzyl-isobuttersäure
α-(4-Chlor-benzyl)-isobuttersäure
α-(4-Methoxy-benzyl)-isobuttersäure
α,α-Dimethyl-cyanessigsäure
Cyclopropan-carbonsäure
1-Methyl-cyclopropan-carbonsäure
2,2-Dichlor-1-methyl-cyclopropan-carbonsäure
Cyclopentan-carbonsäure
1-Methyl-cyclopentan-carbonsäure
Cyclohexan-carbonsäure
1-Methyl-cyclohexan-carbonsäure
1-Methyl-4-isopropyl-cyclohexan-carbonsäure
3-Methyl-oxetan-3-carbonsäure
2-Methyl-furfuryl-2-carbonsäure
2-Methyl-tetrahydropropan-2-carbonsäure

Die Carbonsäure-Derivate der Formel (III) sind bekannt oder lassen sich nach im Prinzip bekannten Methoden in einfacher Weise herstellen.

So erhält man beispielsweise asymmetrische Carbonsäureanhydride der Verbindungen der Formel (III), wenn man Carbonsäuren (d.h. Y = Hydroxy in Formel (III)) mit Kohlensäurealkyl- oder -aryl-ester-chloriden (Alkyl-O-CO-Cl bzw. Aryl-O-CO-Cl) bzw. mit Alkyl-oder Arylsulfonsäurechloriden in Gegenwart eines Verdünnungsmittels, wie z.B. Methylenchlorid, und in Gegenwart eines Säurebindemittels, wie z.B. Triethylamin oder Pyridin, bei Temperaturen zwischen -20°C und +100°C, vorzugsweise zwischen 0°C und 50°C, umsetzt.

Die asymmetrischen Säureanhydride der Formel (III) werden im allgemeinen nicht in reiner Form isoliert, sondern in der anfallenden Form, gegebenenfalls nach vorheriger Entfernung von Verdünnungsmittel, und/oder als Salze weiter verwendet.

Verwendet man die entsprechenden Carbonsäurehalogenide, so kommen als Säurebindemittel bei der Umsetzung nach dem erfindungsgemäßen Verfahren (a) alle üblichen Säureakzeptoren in Betracht. Vorzugsweise verwendbar sind tertiäre Amine, wie Triethylamin, Pyridin und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat. Es ist auch möglich, die jeweiligen Anilin-Derivate der

Formel (II) gleichzeitig als Säurebindemittel zu verwenden. Dazu muß die betreffende Anilin-Verbindung dann zumindest in solcher Menge eingesetzt werden, daß der freiwerdende Halogenwasserstoff gebunden werden kann.

Als Verdünnungsmittel können bei dem erfindungsgemäßen Verfahren (a) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Bei Verwendung der Säurehalogenide als Carbonsäure-Derivate der Formel (III) können die Reaktionstemperaturen bei der Durchführung des erfindungsgemäßen Verfahrens (a) innerhalb eines größeren Bereiches variiert werden. Arbeitet man ohne Lösungsmittel und Säurebindemittel, so geht man im allgemeinen so vor, daß man die Komponenten zunächst bei Temperaturen zwischen -20° C und +20° C reagieren läßt und danach auf Temperaturen zwischen 70° C und 200° C erhitzt. Arbeitet man in Gegenwart eines Verdünnungsmittels und eines Säurebindemittels, so liegen die Reaktionstemperaturen im allgemeinen zwischen -20° C und +100° C, vorzugsweise zwischen 0° C und 50° C.

Das erfindungsgemäße Verfahren (a) wird im allgemeinen unter Normaldruck durchgeführt.

Bei der Durchführung des erfindungsgemäßen Verfahrens (a) werden die Ausgangsstoffe der Formel (II) und das entsprechende Säurehalogenid der Formel (III) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem mit Wasser wenig mischbaren organischen Lösungsmittel extrahiert, die organische Phase wäscht, einengt und den verbleibenden Rückstand gegebenenfalls üblichen Reinigungsverfahren unterwirft.

Verwendet man bei dem erfindungsgemäßen Verfahren (a) als Reaktionskomponente der Formel (III) symmetrische oder asymmetrische Carbonsäure-anhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können auch bei dem erfindungsgemäßen Verfahren (a) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (a) werden die Ausgangsstoffe der Formel (II) und das Carbonsäureanhydrid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Analog zu der bei der Verwendung von Carbonsäureanhydriden als Reaktionskomponente der Formel (III) beschriebenen Verfahrensweise, erfolgt auch die Umsetzung mit solchen Carbonsäure-Derivaten der Formel (III), in welcher Y für Alkylsulfonyloxy, Arylsulfonyloxy oder die Gruppe

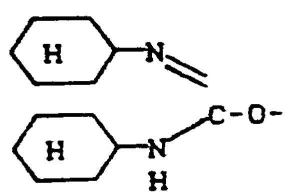

steht.

Die bei dem erfindungsgemäßen Verfahren (b) als Ausgangsstoffe benötigten Pyridin-Derivate der Formel (IV) wurden bereits im Zusammenhang mit der Beschreibung des Verfahrens (A) abgehandelt.

Die bei dem erfindungsgemäßen Verfahren (b) weiterhin als Ausgangsstoffe benötigten Acylanilin-Derivate sind durch die Formeln (Va) und (Vb) eindeutig definiert. In diesen Formeln haben $R^4$, $R^5$, $R^6$, $R^7$, $R^8$, X und n vorzugsweise diejenigen Bedeutungen, die bereits im Zusammenhang mit der Beschreibung der Stoffe der Formel (I) vorzugsweise für diese Reste bzw. für diesen Index genannt wurden.

Die Verbindungen der Formeln (Va) und (Vb) sind bekannt oder lassen sich nach im Prinzip bekannten Methoden in einfacher Weise herstellen. So erhält man Acylanilin-Derivate der Formel (Va) z.B. dadurch, daß man 4-Aminothiophenole der Formel

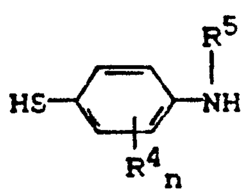

(IX)

in welcher

$R^4$, $R^5$ und n die oben angegebene Bedeutung haben,

mit Carbonsäurederivaten der Formel

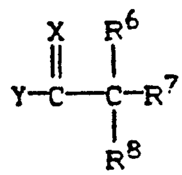

(III)

in welcher

$R^6$, $R^7$, $R^8$, X und Y die oben angegebene Bedeutung haben,

gegebenenfalls in Gegenwart eines Verdünnungsmittels und gegebenenfalls in Gegenwart eines Säurebindemittels umsetzt. Die Reaktionsbedingungen entsprechen dabei denjenigen, die auch bei der Durchführung des Verfahrens (a) angewandt werden.

Als Säurebindemittel können bei der Durchführung des erfindungsgemäßen Verfahrens (b) alle üblichen Säureakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, ferner Alkalimetall- und Erdalkalimetall-amide und -hydride, wie Natriumamid, Natriumhydrid und Calciumhydrid, und außerdem auch Alkalimetall-alkoholate, wie Natrium-methylat, Natriumethylat und Kalium-tert.-butylat.

Als Verdünnungsmittel können bei dem erfindungsgemäßen Verfahren (b) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem Nitrile, wie Acetonitril und Propionitril, und weiterhin polare Lösungsmittel, wie Nitrobenzol, Dimethylsulfoxid, Sulfolan, Dimethylformamid und N-Methyl-pyrrolidon.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0°C und 200°C, vorzugsweise zwischen 50°C und 150°C.

Das erfindungsgemäße Verfahren (b) wird im allgemeinen unter Normaldruck durchgeführt.

Bei der Durchführung des erfindungsgemäßen Verfahrens (b) setzt man die Reaktionskomponenten der Formeln (IV) und (Va) bzw. (Vb) im allgemeinen in etwa äquimolaren Mengen ein. Es ist jedoch auch möglich, die eine oder andere Komponente in einem größeren Überschuß (bis zu 2 Mol) zu verwenden. Außerdem setzt man im allgemeinen auch eine äquimolare Menge an säurebindendem Mittel ein. Es kann jedoch auch von Vorteil sein, das Säurebindemittel in einem bis zu einmolaren Überschuß hinzuzufügen. Im einzelnen geht man im allgemeinen so vor, daß man das säurebindende Mittel zu einer Mischung der Reaktionskomponenten in einem geeigneten Verdünnungsmittel gibt. Man kann aber auch in der Weise verfahren, daß man zunächst aus dem Acylanilin-Derivat der Formel (Va) bzw. (Vb) und dem Säurebindemittel ein Salz erzeugt und dieses dann mit einem Pyridin-Derivat der Formel (IV) umsetzt. Weiterhin ist es auch möglich, aus dem Acylanilin-Derivat der Formel (Va) bzw. (Vb) mit einem Säurebindemittel zunächst separat ein Salz herzustellen, dieses dann zu isolieren und anschließend in Gegenwart eines geeigneten Verdünnungsmittels ohne weitere Zugabe eines Säurebindemittels mit einem Pyridin-Derivat der Formel (IV) umzusetzen. Die Aufarbeitung erfolgt jeweils nach üblichen Methoden.

Die bei dem erfindungsgemäßen Verfahren (c) als Ausgangsstoffe benötigten Carbonsäureanilide der Formel (I), in welcher $R^5$ für Wasserstoff steht, sind erfindungsgemäße Verbindungen und erhältlich nach Verfahren (a), (b), (c) oder (d). In dieser Formel haben $R^1$, $R^2$, $R^3$, $R^4$, $R^6$, $R^7$, $R^8$, n und X vorzugsweise die oben beschriebene Bedeutung.

Die bei dem erfindungsgemäßen Verfahren (c) weiterhin als Ausgangsstoffe benötigten Alkylierungsmittel sind durch die Formel (VI) definiert. In dieser Formel hat $R^5$ die oben angegebene Bedeutung. Y steht vorzugsweise für Fluor, Chlor, Methyl- oder Ethylsulfonyloxy, Phenyl- oder p-Tolylsulfonyloxy. Die Alkylierungsmittel der Formel (VI) sind bekannt.

In Abhängigkeit von der Art des Substituenten X nimmt bei dem Verfahren (c) die Reaktion zwischen den Reaktionskomponenten einen unterschiedlichen Verlauf. Stellt X ein Sauerstoffatom dar, so erfolgt der Eintritt des Restes $R^5$ bevorzugt am N-Atom der Carbonamidgruppe unter Bildung der Verbindungen vom Typ (Ia). Stellt X ein Schwefelatom dar, so erfolgt der Eintritt des Restes $R^5$ (entspricht dann $R^9$) am Schwefelatom der Thioamidgruppe unter Bildung der Verbindungen vom Typ (Ib).

Die Umsetzungen gemäß dem Verfahren (c) werden vorzugsweise in Verdünnungsmitteln durchgeführt. Als solche sind geeignet aliphatische und aromatische Kohlenwasserstoffe, wie Hexan oder Toluol, Ether, wie Diethylether, Tetrahydrofuran oder Dioxan, Acetonitril, Dimethylsulfoxid u.a., im Falle des Einsatzes von Thioamiden auch Alkohole, wie Methanol und Ethanol.

Weiterhin werden die Umsetzungen gemäß Verfahren (c) vorzugsweise in Gegenwart einer säurebindenden Base vorgenommen. Als solche sind geeignet: Alkali- und Erdalkalioxide, -hydroxide und -carbonate, Alkalialkoholate, -amide oder -hydride.

Die Reaktionskomponenten und das säurebindende Mittel werden vorzugsweise im stöchiometrischen Verhältnis zueinander eingesetzt, jedoch kann das säurebindende Mittel und die Reaktionskomponente der Formel (VI) auch im Überschuß bis zu einem weiteren Mol eingesetzt werden. Die Reaktionstemperaturen betragen im allgemeinen -50° C bis +200° C, vorzugsweise 0° C bis 100° C.

Die bei dem erfindungsgemäßen Verfahren (d) als Ausgangsstoffe benötigten Carbonsäure-imidhalogenide sind durch die Formel (VII) definiert. In dieser Formel haben $R^1$, $R^2$, $R^3$, $R^4$, $R^6$, $R^7$, $R^8$ und n vorzugsweise die bei der Beschreibung der erfindungsgemäßen Stoffe der Formel (I) für diese Reste vorzugsweise angegebene Bedeutung, $Hal^2$ steht für Chlor oder Brom. Die Carbonsäure-imidhalogenide der

Formel (VII) sind bisher nicht bekannt. Man erhält sie aus den erfindungsgemäßen Pyridylthioacylaniliden der Formel (I), in denen $R^5$ für Wasserstoff und X für Sauerstoff steht, indem diese unter grundsätzlich bekannten Bedingungen mit anorganischen Säurechloriden, wie Phosphor(V)-chlorid, Phosphoroxychlorid, Thionylchlorid, Phosgen oder Dihalogen-phosphanen umgesetzt werden.

Die bei dem erfindungsgemäßen Verfahren (d) weiterhin als Ausgangsstoffe benötigten Nucleophile sind durch die Formel (VIII) definiert, worin X ein Sauerstoff- oder Schwefelatom oder den Rest $N-R^{10}$ oder $N-O-R^{11}$ bedeuten mit der gleichen Bedeutung für $R^9$, $R^{10}$ und $R^{11}$ wie oben angegeben. Demgemäß handelt es sich um Alkohole, Mercaptane, Ammoniak, primäre und sekundäre Amine sowie Hydroxylamin bzw. dessen am N- oder O-Atom entsprechend substituierte Derivate. Diese Verbindungen sind literaturbekannt.

Die Umsetzung gemäß Verfahren (d) wird vorzugsweise in Verdünnungsmitteln und in Gegenwart eines säurebindenden Mittels durchgeführt.

Als Verdünnungsmittel sind geeignet: Kohlenwasserstoffe und Halogenkohlenwasserstoffe wie Dichlormethan, Chloroform oder Toluol, Ether, wie Diethylether, Tetrahydrofuran oder Dioxan. Im Falle von Alkoholen als Reaktionskomponente können diese im Überschuß auch als Verdünnungsmittel verwendet werden.

Als säurebindende Mittel sind geeignet: Alkali- und Erdalkali-oxide, -hydroxide und -carbonate, sowie tertiäre

Amine, wie Triethylamin und Pyridin. Im Falle des Ammoniaks, primärer oder sekundärer Amine als Reaktionskomponente kann ein weiteres Mol von diesen auch als säurebindendes Mittel verwendet werden. Solche säurebindenden Mittel können im Überschuß auch gleichzeitig als Lösungsmittel fungieren.

Die Reaktionskomponenten und das säurebindende Mittel werden bei Verfahren (d) vorzugsweise im äquimolaren Verhältnis eingesetzt, jedoch kann sowohl die nucleophile Reaktionskomponente wie auch das säurebindende Mittel im Überschuß bis zu mehreren (vorzugsweise 5) Molen eingesetzt werden.

Die Reaktionstemperaturen können bei Verfahren (d) in einem größeren Bereich variiert werden. Im allgemeinen arbeitet man bei Temperaturen zwischen -50 und +200° C, vorzugsweise zwischen 0° C und 100° C.

Die bei dem erfindungsgemäßen Verfahren (e) als Ausgangsstoffe benötigten Carbonsäureanilide sind durch die Formel (Ic) definiert. In dieser Formel haben die Substituenten $R^1$, $R^2$, $R^3$, $R^4$, $R^5$, $R^6$, $R^7$, $R^8$ und n vorzugsweise die oben bei der Beschreibung der erfindungsgemäßen Stoffe der Formel (I) für diese Substituenten vorzugsweise angegebene Bedeutung und X steht für ein Sauerstoffatom. Die Verbindungen der Formel (Ic) sind erfindungsgemäße Verbindungen, d.h. sie werden durch die vorliegende Erfindung mit umfaßt und können nach den vorangehend beschriebenen Verfahren hergestellt werden.

Gemäß dem erfindungsgemäßen Verfahren (e) werden die Verbindungen der Formel (Ic) mit X = Sauerstoff in solche der Formel (I) mit X = Schwefel umgewandelt. Reaktionen dieser Art sind grundsätzlich bekannt. Diese werden durchgeführt, indem man auf die Verbindungen der Formel (Ic) Schwefelungsreagentien wie $P_4S_{10}$ oder 2,4-Bis-(4-methoxyphenyl)-2,4-dithiono-1,3,2,4-dithiaphosphetan (Lawesson-Reagens), einwirken läßt.

Die Umsetzung gemäß Verfahren (e) wird vorzugsweise in Gegenwart eines Verdünnungsmittels durchgeführt. Als solche sind inerte organische Verdünnungsmittel geeignet. Vorzugsweise verwendet man Toluol, Xylol und Benzol.

0243868

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich besonders gut zur selektiven Bekämpfung mono- und dikotyler Unkräuter in monokotylen Kulturen, wie z.B. Mais und Getreide.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlorpyridin-2-oxy)-phenoxy]-propionsäure(trimethylsilyl)-methylesters, das R-Enantiomere des 2-[4-(3,5-Dichlorpyridyl-2-oxy)-phenoxy]-propionsäure(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Diiod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

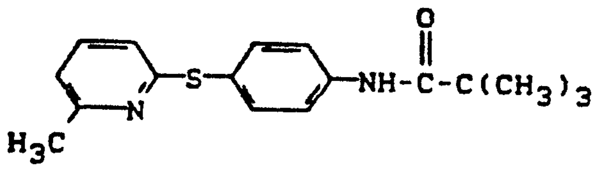

Verfahren (a)

21,6 g (0,1 Mol) 4-(6-Methyl-pyridyl-2-thio)-anilin und 10,1 g (0,1 Mol) Triethylamin werden in 150 ml Tetrahydrofuran gelöst. Hierzu tropft man bei 10°C bis 15°C 12,05 g (0,1 Mol) Pivalsäurechlorid zu. Die Mischung wird 2 Stunden bei Raumtemperatur nachgerührt und anschließend in 1 l Wasser eingegossen. Die abgeschiedenen Kristalle werden abgesaugt und an der Luft getrocknet.

Man erhält 26,1 g (87 % der Theorie) Pivalinsäure-4-(6-methyl-pyridyl-2-thio)-anilid vom Schmelzpunkt 170°C bis 171°C (umkristallisiert aus Toluol).

### Beispiel 2

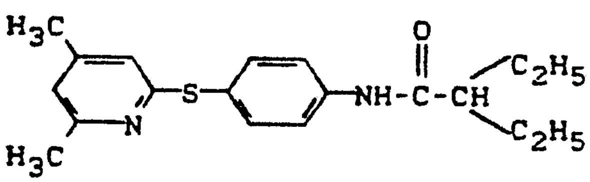

Verfahren (a)

23,0 g (0,1 Mol) 4-(4,6-Dimethyl-pyridyl-2-thio)-anilin werden mit 23,0 g (0,2 Mol) α-Ethyl-buttersäureanhydrid 5 Stunden unter Rückfluß erhitzt. Das Gemisch wird im Vakuum eingedampft, der Rückstand in 1 l Wasser eingerührt. Die abgeschiedenen Kristalle werden abgesaugt und an der Luft getrocknet.

Man erhält 26,9 g (82 % der Theorie) Pentan-3-carbonsäure-4-(4,6-dimethyl-pyridyl-2-thio)-anilid vom Schmelzpunkt 152°C bis 154°C (umkristallisiert aus Waschbenzin).

Beispiel 3

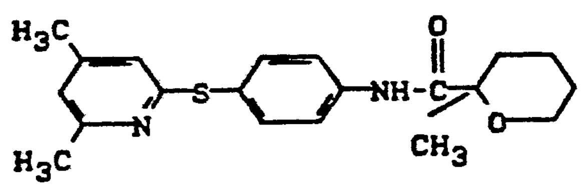

Verfahren (a)

14,4 g (0,1 Mol) 2-Methyl-pyran-2-carbonsäure und 10,1 g (0,1 Mol) Triethylamin werden in 100 ml Tetrahydrofuran gelöst. Hierzu tropft man unter Kühlung bei 5°C bis 10°C 10,85 g (0,1 Mol) Chlorameisensäureethylester zu und rührt das Gemisch noch weitere 2 Stunden bei Raumtemperatur. Dann werden 23,0 g (0,1 mol) 4-(4,6-Dimethyl-pyridyl-2-thio)-anilin eingetragen. Das Gemisch wird 1 Stunde bei Raumtemperatur nachgerührt, 2 Stunden unter Rückfluß gekocht und nach dem Abkühlen in 1 l Eiswasser eingerührt. Die abgeschiedenen Kristalle werden abgesaugt und an der Luft getrocknet.

Man erhält 27,4 g (77 % der Theorie) 2-Methyl-pyran-2-carbonsäure-4-(4,6-dimethyl-pyridyl-2-thio)-anilid vom Schmelzpunkt 107° C bis 109° C (umkristallisiert aus Waschbenzin).

Beispiel 4

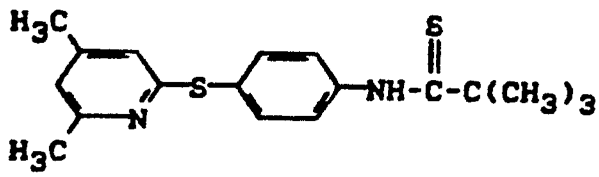

23,89 g (76 mmol) Pivalinsäure-4-(4,6-dimethyl-pyridyl-2-thio)-anilid und 16,72 g (41,8 mmol) Lawesson-Reagenz werden in 76 ml absolutem Toluol unter DC-Kontrolle unter Rückfluß erhitzt. Das Gemisch wird auf Raumtemperatur abgekühlt und an Kieselgel mit Toluol/Aceton 20:1 filtriert. Die Lösung wird eingedampft, der Rückstand aus Essigester/n-Hexan kristallisiert.

Man erhält 15,98 g (63,6 % der Theorie) Pivaloylthio-4-(4,6-dimethyl-pyridyl-2-thio)-anilid vom Schmelzpunkt 127° C.

In entsprechender Weise und gemäß den allgemeinen Angaben zur Herstellung erhält man die in der folgenden Tabelle 2 aufgeführten Verbindungen der Formel (Ia) bzw. (Ib):

Tabelle 2

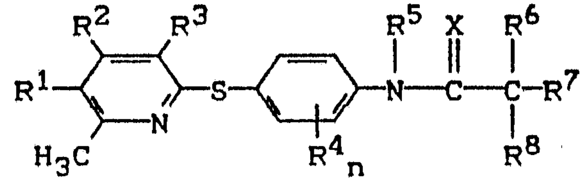

(Ia)

| Bsp. Nr. | $R^1$ | $R^2$ | $R^3$ | $R^4$ | n | $R^5$ | X | $R^6$ | $R^7$ | $R^8$ | Fp./°C |
|---|---|---|---|---|---|---|---|---|---|---|---|
| 5 | H | H | H | - | 0 | H | O | $CH_3$ | $CH_3$ | $CH_3$ | 170-172 |
| 6 | H | H | H | - | 0 | H | O | $CH_2Cl$ | $CH_3$ | $CH_3$ | 138-140 |
| 7 | H | H | H | - | 0 | H | O | $CH_3$ | $-(CH_2)_4-$ | | 124-126 |
| 8 | H | $CH_3$ | H | - | 0 | H | O | H | $C_2H_5$ | $C_2H_5$ | 152-154 |
| 9 | H | $CH_3$ | H | - | 0 | H | O | $CH_3$ | $CH_3$ | $CH_3$ | 156-157 |
| 10 | H | $CH_3$ | H | - | 0 | H | O | $CH_2Cl$ | $CH_3$ | $CH_3$ | 144-145 |
| 11 | H | $CH_3$ | H | - | 0 | H | O | $C_2H_5$ | $CH_3$ | $CH_3$ | 137-139 |
| 12 | H | $CH_3$ | H | - | 0 | H | O | $C_3H_7$ | $CH_3$ | $CH_3$ | 75- 76 |
| 13 | H | $CH_3$ | H | - | 0 | H | O | $CH(CH_3)_2$ | $CH_3$ | $CH_3$ | 130-132 |
| 14 | H | $CH_3$ | H | - | 0 | H | O | CN | $CH_3$ | $CH_3$ | 134-136 |
| 15 | H | $CH_3$ | H | - | 0 | H | O | $CH_3$ | $-(CH_2)_5-$ | | 108-109 |
| 16 | H | $CH_3$ | H | - | 0 | H | O | $-O-(CH_2)_4-$ | | $CH_3$ | 107-109 |
| 17 | H | $CH_3$ | CN | - | 0 | H | O | $CH_3$ | $CH_3$ | $CH_3$ | 153-155 |
| 18 | H | $CH_3$ | CN | - | 0 | H | O | $C_2H_5$ | $CH_3$ | $CH_3$ | 174-176 |
| 19 | H | $CH_3$ | CN | - | 0 | H | O | $CH_3$ | $-(CH_2)_5-$ | | 128-130 |
| 20 | H | $CH_3$ | CN | 3-Cl | 1 | H | O | $CH_3$ | $CH_3$ | $CH_3$ | 162-164 |
| 21 | H | $CH_3$ | H | - | 0 | H | S | $CH_3$ | $CH_3$ | $C_2H_5$ | 76 |
| 22 | H | $CH_3$ | H | - | 0 | H | S | $CH_3$ | $CH_3$ | $i-C_3H_7$ | 99 |

Beispiel 23

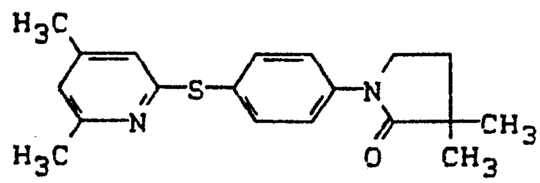

Schmelzpunkt: 134° C

Beispiel 24

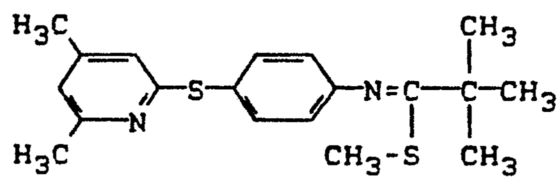

Verfahren (c)

4,96 g (15 mMol) Pivaloylthio-4-(4,6-dimethyl-pyridyl-2-thio)-anilid werden in 60 ml tert.-Butanol bei 50° C mit 2,02 g (18 mMol) Kalium-tert.-butylat versetzt, 15 Minuten verrührt und nach Zugabe von 1,1 ml (18 mMol) Methyliodid unter Kontrolle von Dünnschichtchromatographie gerührt. Die Reaktionsmischung wird in 200 ml Wasser eingerührt und das Reaktionsprodukt mit Essigsäureethylester extrahiert. Die organische Phase wird getrocknet und das Lösungsmittel im Vakuum abdestilliert. Der Rückstand wird mit Cyclohexan/Essigsäureethylester (3:1) chromatographiert (Säulenchromatographie) und in n-Hexan umkristallisiert.
Man erhält 3,6 g (69,7 % der Theorie) des gewünschten Produktes vom Schmelzpunkt 78° C.

Beispiel 25

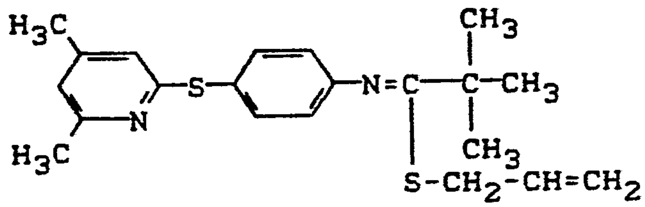

Schmelzpunkt: 43° C

Herstellung erfolgt analog zu Beispiel 24.

0243868

Herstellung der Ausgangsprodukte

Beispiel II-1

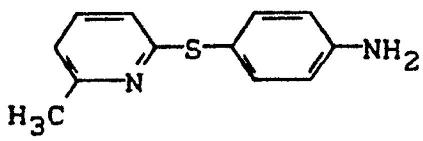

12,5 g (0,1 Mol) 4-Amino-thiophenol werden in 50 ml Tetramethylensulfon gelöst. Hierzu gibt man portionsweise 5,6 g (0,1 Mol) pulverisiertes Kaliumhydroxid. Die Mischung wird 30 Minuten bei Raumtemperatur verrührt, anschließend mit 12,75 g (0,1 Mol) 2-Chlor-6-methyl-pyridin versetzt und dann 5 Stunden auf 120° C bis 130° C erhitzt. Nach dem Abkühlen gießt man das Reaktionsgemisch in 1 l Eiswasser. Die abgeschiedenen Kristalle werden abgesaugt und an der Luft getrocknet.

Man erhält 17,8 g (82,4 % der Theorie) 4-(6-Methyl-pyridyl-2-thio)-anilin vom Schmelzpunkt 72° C bis 74° C (umkristallisiert aus Tetrachlorkohlenstoff).

In entsprechender Weise gewinnt man:

II-2: 4-(4,6-Dimethyl-pyridyl-2-thio)-anilin; Fp. 108° C bis 110° C (umkristallisiert aus Tetrachlorkohlenstoff)

II-3: 4-(4,6-Dimethyl-3-cyano-pyridyl-2-thio)-anilin; Fp. 144° C bis 146° C (umkristallisiert aus Toluol)

II-4: 3-Chlor-4-(4,6-dimethyl-3-cyano-pyridyl-2-thio)-anilin; Fp. 163° C bis 165° C (umkristallisiert aus Toluol).

## Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine sehr gute herbizide Wirkung insbesondere in Amaranthus und Chenopodium bei sehr guter Nutzpflanzenverträglichkeit, insbesondere in Mais und Getreide, zeigen in diesem Test beispielsweise die Verbindungen gemäß den Herstellungsbeispielen 1, 9, 10, 12, 13 und 14.

## Patentansprüche

1. Pyridylthio-acylanilide der Formel (I),

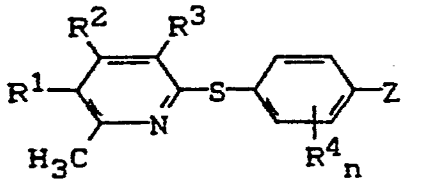

(I)

in welcher

$R^1$, $R^2$ und $R^3$ unabhängig voneinander für Wasserstoff, Halogen, Cyano, Trifluormethyl, für Alkyl, Alkoxy und Alkylthio mit jeweils 1 bis 4 Kohlenstoffatomen stehen,

$R^4$ für Halogen, Methyl oder Methoxy steht,

n für eine Zahl 0, 1 oder 2 steht,

Z für die Gruppe (Ia)

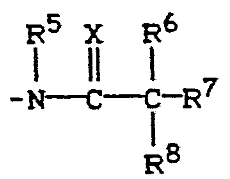

(Ia)

oder die Gruppe (Ib)

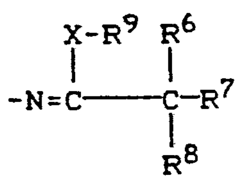

(Ib)

steht,

wobei

X für Sauerstoff, Schwefel, eine Gruppe $N-R^{10}$ oder $N-O-R^{11}$ steht, wobei

$R^{10}$ und $R^{11}$ unabhängig voneinander für Wasserstoff oder gegebenenfalls substituiertes Alkyl mit 1 bis 6 Kohlenstoffatomen, für Alkenyl oder Alkinyl mit jeweils 2 bis 6 Kohlenstoffatomen stehen,

$R^5$ für Wasserstoff oder gegebenenfalls substituiertes Alkyl mit 1 bis 6 Kohlenstoffatomen oder jeweils gegebenenfalls substituiertes Alkenyl oder Alkinyl mit jeweils 2 bis 6 Kohlenstoffatomen steht,

$R^6$ für Wasserstoff, Halogen, Cyano, für gegebenenfalls substituiertes Alkyl mit 1 bis 6 Kohlenstoffatomen, Alkenyl mit 2 bis 6 Kohlenstoffatomen, jeweils gegebenenfalls substituiertes Aryl oder Aralkyl oder für die Reste $-OR^{12}$ oder $-S(O)_m-R^{12}$ steht, wobei

$R^{12}$ für gegebenenfalls substituiertes Alkyl mit 1 bis 6 Kohlenstoffatomen oder für gegebenenfalls substituiertes Aryl steht und

m für eine Zahl 0, 1 oder 2 steht,

$R^7$ und $R^8$ unabhängig voneinander für Halogen oder für gegebenenfalls substituiertes Alkyl mit 1 bis 6 Kohlenstoffatomen stehen,

$R^9$ für gegebenenfalls substituiertes Alkyl mit 1 bis 6 Kohlenstoffatomen steht, für Alkenyl oder Alkinyl mit jeweils 3 bis 6 Kohlenstoffatomen steht oder

$R^6$ und $R^7$ bzw. $R^6$ und $R^8$ gemeinsam mit dem angrenzenden Kohlenstoffatom für einen gegebenenfalls substituierten, gesättigten oder ungesättigten Ring mit 3 bis 8 Ringatomen stehen, der außer Kohlenstoff- auch Sauerstoff- und Schwefelatome als Ringglieder enthalten kann, oder

$R^5$ und $R^7$ bzw. $R^5$ und $R^{10}$ bzw. $R^7$ und $R^9$ bzw. $R^8$ und $R^9$ bzw. $R^7$ und $R^8$ gemeinsam für eine Alkylenkette mit 2 bis 6 Kohlenstoffatomen oder eine Alkenylenkette mit 2 bis 6 Kohlenstoffatomen stehen, welche jeweils durch Halogen, $C_1$-$C_4$-Alkyl oder $C_1$-$C_4$-Alkoxy substituiert sein können, oder

$R^5$ und $R^{11}$ gemeinsam für eine Alkylenkette mit 1 bis 5 Kohlenstoffatomen oder eine Alkenylenkette mit 2 bis 5 Kohlenstoffatomen stehen, welche jeweils durch Halogen, $C_1$-$C_4$-Alkyl oder $C_1$-$C_4$-Alkoxy substituiert sein können oder

X und $R^9$ gemeinsam für den Rest 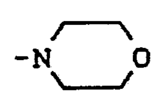 stehen.

2. Pyridylthio-acylanilide der Formel (I) gemäß Anspruch 1, in welcher

$R^1$, $R^2$ und $R^3$ unabhängig voneinander für Wasserstoff, Fluor, Chlor, Brom, Cyano, Trifluormethyl, für Alkyl, Alkoxy oder Alkylthio mit jeweils 1 bis 4 Kohlenstoffatomen stehen,

$R^4$ für Fluor, Chlor, Brom, Methyl oder Methoxy steht,

n für eine Zahl 0, 1 oder 2 steht,

Z für die Gruppe (Ia)

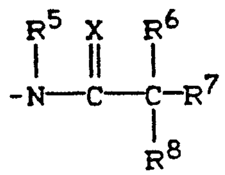

(Ia)

oder die Gruppe (Ib)

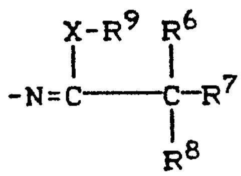

(Ib)

steht,

wobei

X für Sauerstoff, Schwefel, eine Gruppe N-$R^{10}$ oder N-O-$R^{11}$ steht, wobei

$R^{10}$ und $R^{11}$ unabhängig voneinander für Wasserstoff oder Alkyl mit 1 bis 4 Kohlenstoffatomen, für Alkenyl oder Alkinyl mit jeweils 2 bis 4 Kohlenstoffatomen stehen,

$R^5$ für Wasserstoff oder für Alkyl mit 1 bis 4 Kohlenstoffatomen, Alkenyl oder Alkinyl mit jeweils 2 bis 6 Kohlenstoffatomen steht,

$R^6$ für Wasserstoff, Fluor, Chlor, Brom, Cyano, für gegebenenfalls einfach oder mehrfach, gleich oder verschieden durch Fluor, Chlor und Brom substituiertes Alkyl mit 1 bis 4 Kohlenstoffatomen, Alkenyl mit 2 bis 4 Kohlenstoffatomen, für jeweils gegebenenfalls einfach bis fünffach, gleich oder verschieden durch Fluor, Chlor, Brom, Trifluormethyl, Methyl und Methoxy substituiertes Phenyl oder Benzyl oder für die Reste -$OR^{12}$ oder -$S(O)_m$-$R^{12}$ steht, wobei

$R^{12}$ für gegebenenfalls einfach oder mehrfach, gleich oder verschieden durch Fluor, Chlor, Brom, Methoxy oder Ethoxy substituiertes Alkyl mit 1 bis 4 Kohlenstoffatomen oder für

gegebenenfalls einfach bis fünffach, gleich oder verschieden durch Fluor, Chlor, Brom und $C_1$-$C_4$-Alkyl substituiertes Phenyl steht und

m für eine Zahl 0, 1 oder 2 steht,

$R^7$ und $R^8$ unabhängig voneinander für Fluor, Chlor, Brom oder für gegebenenfalls einfach oder mehrfach, gleich oder verschieden durch Fluor, Chlor und Brom substituiertes Alkyl mit 1 bis 4 Kohlenstoffatomen stehen,

$R^9$ für gegebenenfalls einfach oder mehrfach, gleich oder verschieden durch Halogen oder $C_1$-$C_4$-Alkoxy substituiertes Alkyl mit 1 bis 4 Kohlenstoffatomen, Alkenyl oder Alkinyl mit jeweils 3 bis 4 Kohlenstoffatomen steht oder

$R^6$ und $R^7$ bzw. $R^6$ und $R^8$ gemeinsam mit dem angrenzenden Kohlenstoffatom für einen gesättigten oder ungesättigten Ring mit 3 bis 7 Ringatomen stehen, der außer Kohlenstoff auch Sauerstoff oder Schwefel enthalten kann, und welcher einfach oder mehrfach, gleich oder verschieden durch Fluor, Chlor oder $C_1$-$C_4$-Alkyl substituiert sein kann, oder

$R^5$ und $R^7$ bzw. $R^5$ und $R^{10}$ bzw. $R^7$ und $R^9$ bzw. $R^8$ und $R^9$ bzw. $R^7$ und $R^8$ gemeinsam für eine Alkylenkette mit 2 bis 5 Kohlenstoffatomen oder eine Alkenylenkette mit 2 bis 5 Kohlenstoffatomen stehen, welche jeweils einfach oder mehrfach, gleich oder verschieden durch Halogen, $C_1$-$C_4$-Alkyl oder $C_1$-$C_4$-Alkoxy substituiert sein können, oder

$R^5$ und $R^{11}$ gemeinsam für eine Alkylenkette mit 1 bis 4 Kohlenstoffatomen oder eine Alkenylenkette mit 2 bis 4 Kohlenstoffatomen stehen, welche jeweils einfach oder mehrfach, gleich oder verschieden durch Halogen oder $C_1$-$C_4$-Alkyl substituiert sein können, oder

X und $R^9$ gemeinsam für den Rest 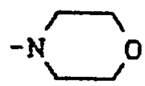 stehen.

3. Pyridylthio-acylanilide der Formel (I) gemäß Anspruch 1, in welcher

$R^1$, $R^2$ und $R^3$ unabhängig voneinander für Wasserstoff, Chlor, Cyano, Trifluormethyl, Methyl, Ethyl oder Methoxy stehen,

$R^4$ für Fluor, Chlor, Methyl oder Methoxy steht,

n für eine Zahl 0 oder 1 steht,

Z für die Gruppe (Ia)

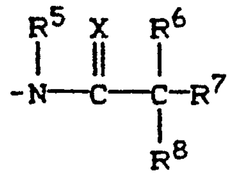 (Ia)

oder die Gruppe (Ib)

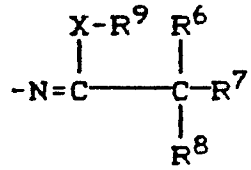 (Ib)

steht,

wobei

X für Sauerstoff, Schwefel, eine Gruppe N-$R^{10}$ oder N-O-$R^{11}$ steht, wobei

$R^{10}$ und $R^{11}$ unabhängig voneinander für Wasserstoff oder für Alkyl mit 1 bis 3 Kohlenstoffatomen oder für Alkenyl oder Alkinyl mit jeweils 2 oder 3 Kohlenstoffatomen stehen,

$R^5$ für Wasserstoff oder für Alkyl mit 1 bis 3 Kohlenstoffatomen, Alkenyl mit 2 bis 4 Kohlenstoffatomen, oder Alkinyl mit 3 bis 4 Kohlenstoffatomen steht,

$R^6$ für Wasserstoff, Fluor, Chlor, Brom, Cyano, für gegebenenfalls einfach bis fünffach, gleich oder verschieden durch Fluor und Chlor substituiertes Alkyl mit 1 bis 4 Kohlenstoffatomen, für jeweils gegebenenfalls einfach bis dreifach, gleich oder verschieden durch Fluor, Chlor, Trifluormethyl und Methyl substituiertes Phenyl oder Benzyl oder für die Reste $-OR^{12}$ oder $-S(O)_m-R^{12}$ steht, wobei

$R^{12}$ für gegebenenfalls einfach bis fünffach, gleich oder verschieden durch Fluor und Chlor substituiertes Alkyl mit 1 bis 4 Kohlenstoffatomen oder für gegebenenfalls einfach bis dreifach, gleich oder verschieden durch Fluor, Chlor und $C_1$-$C_2$-Alkyl substituiertes Phenyl steht und

m für eine Zahl 0, 1 oder 2 steht,

$R^7$ und $R^8$ unabhängig voneinander für Fluor, Chlor oder für gegebenenfalls einfach bis fünffach, gleich oder verschieden durch Fluor und Chlor substituiertes Alkyl mit 1 bis 2 Kohlenstoffatomen stehen,

$R^9$ für gegebenenfalls einfach bis fünffach, gleich oder verschieden durch Halogen oder $C_1$-$C_4$-Alkoxy substituiertes Alkyl mit 1 bis 2 Kohlenstoffatomen oder für Alkenyl mit 3 oder 4 Kohlenstoffatomen steht oder

$R^6$ und $R^7$ bzw. $R^6$ und $R^8$ gemeinsam mit dem angrenzenden Kohlenstoffatom für einen gesättigten oder ungesättigten Ring mit 3 bis 6 Ringatomen stehen, der außer Kohlenstoff auch Sauerstoff und Schwefel enthalten kann, und welcher gegebenenfalls einfach bis dreifach, gleich oder verschieden durch Fluor, Chlor, Methyl, Ethyl, n-Propyl und i-Propyl substituiert sein kann, oder

$R^7$ und $R^8$ gemeinsam für eine Alkylenkette mit 2 bis 5 Kohlenstoffatomen stehen, welche einfach bis fünffach, gleich oder verschieden durch Fluor, Chlor, Methyl, Ethyl oder Methoxy substituiert sein kann, oder

$R^5$ und $R^7$ bzw. $R^5$ und $R^{10}$ bzw. $R^7$ und $R^9$ bzw. $R^8$ und $R^9$ gemeinsam für eine Alkylenkette mit 2 bis 3 Kohlenstoffatomen oder eine Alkenylenkette mit 3 bis 4 Kohlenstoffatomen stehen, welche jeweils einfach bis dreifach, gleich oder verschieden durch Fluor, Chlor, Methyl, Ethyl und Methoxy substituiert sein können, oder

$R^5$ und $R^{11}$ gemeinsam für eine Alkylenkette mit 1 oder 2 Kohlenstoffatomen stehen, welche einfach bis dreifach, gleich oder verschieden durch Methyl oder Ethyl substituiert sein kann.

4. Verfahren zur Herstellung von Pyridylthio-acylaniliden der Formel (I),

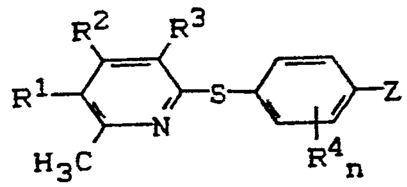

(I)

in welcher

$R^1$, $R^2$ und $R^3$ unabhängig voneinander für Wasserstoff, Halogen, Cyano, Trifluormethyl, für Alkyl, Alkoxy und Alkylthio mit jeweils 1 bis 4 Kohlenstoffatomen stehen,

$R^4$ für Halogen, Methyl oder Methoxy steht,

n für eine Zahl 0, 1 oder 2 steht,

Z für die Gruppe (Ia)

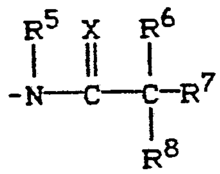

(Ia)

oder die Gruppe (Ib)

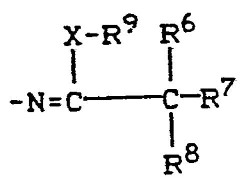

(Ib)

steht,

wobei

X für Sauerstoff, Schwefel, eine Gruppe N-$R^{10}$ oder N-O-$R^{11}$ steht, wobei

$R^{10}$ und $R^{11}$ unabhängig voneinander für Wasserstoff oder gegebenenfalls substituiertes Alkyl mit 1 bis 6 Kohlenstoffatomen, für Alkenyl oder Alkinyl mit jeweils 2 bis 6 Kohlenstoffatomen stehen,

$R^5$ für Wasserstoff oder gegebenenfalls substituiertes Alkyl mit 1 bis 6 Kohlenstoffatomen oder jeweils gegebenenfalls substituiertes Alkenyl oder Alkinyl mit jeweils 2 bis 6 Kohlenstoffatomen steht,

$R^6$ für Wasserstoff, Halogen, Cyano, für gegebenenfalls substituiertes Alkyl mit 1 bis 6 Kohlenstoffatomen, Alkenyl mit 2 bis 6 Kohlenstoffatomen, jeweils gegebenenfalls substituiertes Aryl oder Aralkyl oder für die Reste -O$R^{12}$ oder -$S(O)_m$-$R^{12}$ steht, wobei

$R^{12}$ für gegebenenfalls substituiertes Alkyl mit 1 bis 6 Kohlenstoffatomen oder für gegebenenfalls substituiertes Aryl steht und

m für eine Zahl 0, 1 oder 2 steht,

$R^7$ und $R^8$ unabhängig voneinander für Halogen oder für gegebenenfalls substituiertes Alkyl mit 1 bis 6 Kohlenstoffatomen stehen,

$R^9$ für gegebenenfalls substituiertes Alkyl mit 1 bis 6 Kohlenstoffatomen steht, für Alkenyl oder Alkinyl mit jeweils 3 bis 6 Kohlenstoffatomen steht oder

$R^6$ und $R^7$ bzw. $R^6$ und $R^8$ gemeinsam mit dem angrenzenden Kohlenstoffatom für einen gegebenenfalls substituierten, gesättigten oder ungesättigten Ring mit 3 bis 8 Ringatomen stehen, der außer Kohlenstoff- auch Sauerstoff- und Schwefelatome als Ringglieder enthalten kann, oder

$R^5$ und $R^7$ bzw. $R^5$ und $R^{10}$ bzw. $R^7$ und $R^9$ bzw. $R^8$ und $R^9$ bzw. $R^7$ und $R^8$ gemeinsam für eine Alkylenkette mit 2 bis 6 Kohlenstoffatomen oder eine Alkenylenkette mit 2 bis 6 Kohlenstoffatomen stehen, welche jeweils durch Halogen, $C_1$-$C_4$-Alkyl oder $C_1$-$C_4$-Alkoxy substituiert sein können, oder

$R^5$ und $R^{11}$ gemeinsam für eine Alkylenkette mit 1 bis 5 Kohlenstoffatomen oder eine Alkenylenkette mit 2 bis 5 Kohlenstoffatomen stehen, welche jeweils durch Halogen, $C_1$-$C_4$-Alkyl oder $C_1$-$C_4$-Alkoxy substituiert sein können, oder

X und $R^9$ gemeinsam für den Rest 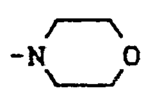 stehen,

dadurch gekennzeichnet, daß man

a) Anilin-Derivate der Formel (II),

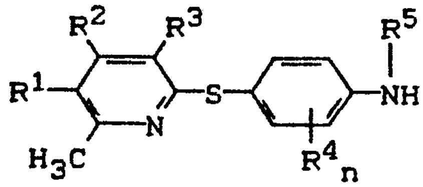

(II)

in welcher

$R^1$, $R^2$, $R^3$, $R^4$, $R^5$ und n die oben angegebene Bedeutung haben,

mit Carbonsäure-Derivaten der Formel (III),

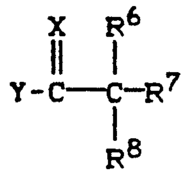

(III)

in welcher

$R^6$, $R^7$, $R^8$ und X die oben angegebene Bedeutung haben und

Y für Hydroxy, Halogen, Acyloxy, Alkoxycarbonyloxy oder Aryloxycarbonyloxy, Alkyl- oder Arylsulfonyloxy oder für die Gruppe

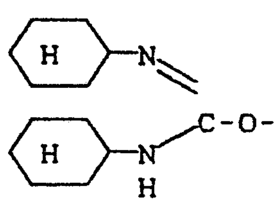

steht,

gegebenenfalls in Gegenwart eines Verdünnungsmittels und gegebenenfalls in Gegenwart eines säurebindenden oder wasserbindenden Mittels umsetzt,

oder daß man

b) Pyridin-Derivate der Formel (IV),

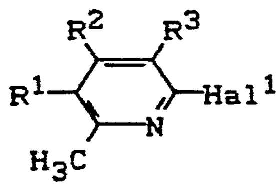

(IV)

in welcher

$R^1$, $R^2$ und $R^3$ die oben angegebene Bedeutung haben und

$Hal^1$ für Halogen steht,

mit Acylanilin-Derivaten der Formel (Va) oder (Vb),

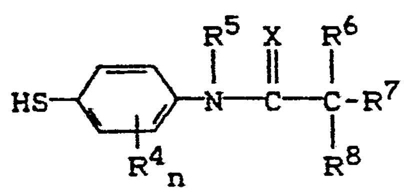

(Va)

oder

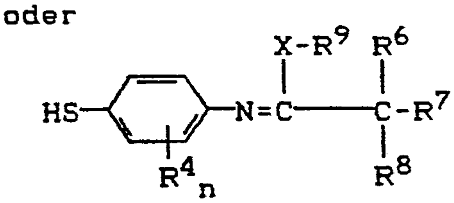

(Vb)

in welcher

$R^4$, $R^5$, $R^6$, $R^7$, $R^8$, $R^9$, X und n die oben angegebene Bedeutung haben,

in Gegenwart eines Verdünungsmittels und gegebenenfalls in Gegenwart eines Säurebindemittels umsetzt,

oder daß man

c) Carbonsäureanilide der Formel (I), in welcher $R^5$ für Wasserstoff steht und $R^1$, $R^2$, $R^3$, $R^4$, $R^6$, $R^7$, $R^8$, X und n die oben angegebene Bedeutung haben,

mit einem Alkylierungsmittel der Formel (VI)

$$R^5\text{-}Y^1 \qquad (VI),$$

in welcher

$R^5$ die oben angegebene Bedeutung besitzt und

$Y^1$ für Halogen, Alkylsulfonyloxy oder Arylsulfonyloxy steht,

gegebenenfalls in Gegenwart einer Base und gegebenenfalls in Gegenwart eines Verdünnungsmittels umsetzt,

oder daß man

d) Carbonsäure-imidhalogenide der Formel (VII),

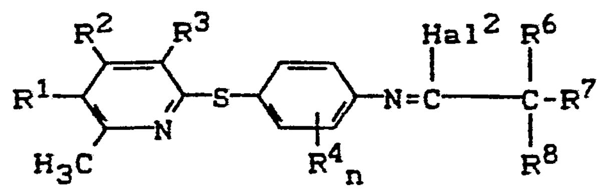

(VII)

in welcher

$R^1$, $R^2$, $R^3$, $R^4$, $R^6$, $R^7$, $R^8$ und n die oben angegebene Bedeutung besitzen und

$Hal^2$ für Chlor oder Brom steht,

mit einem Nucleophil der Formel (VIII),

$HX\text{-}R^9$ (VIII)

in welcher

$R^9$ und X die oben angegebene Bedeutung besitzen,

gegebenenfalls in Gegenwart eines säurebindenden Mittels und gegebenenfalls in Gegenwart eines Lösungsmittels umsetzt,

oder daß man

e) Carbonsäure-anilide der Formel (Ic),

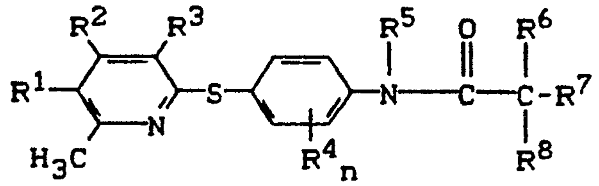

(Ic)

in welcher

$R^1$, $R^2$, $R^3$, $R^4$, $R^5$, $R^6$, $R^7$, $R^8$ und n die oben angegebene Bedeutung besitzen,

mit Schwefelungsreagentien gegebenenfalls in Gegenwart eines Verdünnungsmittels umsetzt.

5. Herbizide Mittel, gekennzeichnet durch einen Gehalt an mindestens einem Pyridylthio-acylanilid der Formel (I) gemäß den Ansprüchen 1 bis 4.

6. Verfahren zur Bekämpfung von Unkräutern, dadurch gekennzeichnet, daß man Pyridylthio-acylanilide der Formel (I) gemäß den Ansprüchen 1 bis 4 auf die Unkräuter und/oder ihren Lebensraum einwirken läßt.

7. Verwendung von Pyridylthio-acylanilide der Formel (I) gemäß den Ansprüchen 1 bis 4 zur Bekämpfung von Unkräutern.

8. Verfahren zur Herstellung von herbiziden Mitteln, dadurch gekennzeichnet, daß man Pyridylthio-acylanilide der Formel (I) gemäß den Ansprüchen 1 bis 4 mit Streckmitteln und/oder oberflächenaktiven Substanzen vermischt.